(19) Europäisches Patentamt
European Patent Office
Office européen des brevets

(11) EP 0 934 319 B1

(12) **FASCICULE DE BREVET EUROPEEN**

(45) Date de publication et mention de la délivrance du brevet: **05.06.2002 Bulletin 2002/23**

(21) Numéro de dépôt: **97943932.0**

(22) Date de dépôt: **03.10.1997**

(51) Int Cl.[7]: **C07D 471/04**, A61K 31/435 // (C07D471/04, 221:00, 209:00)

(86) Numéro de dépôt international: **PCT/FR97/01750**

(87) Numéro de publication internationale: **WO 98/15552 (16.04.1998 Gazette 1998/15)**

(54) **DERIVES DE 1H-PYRIDO[3,4-b]INDOLE-4-CARBOXAMIDE, LEUR PREPARATION ET LEUR APPLICATION EN THERAPEUTIQUE**

1H-PYRIDO[3,4-b]INDOLCARBOXAMIDDERIVATE, IHRE HERSTELLUNG UND THERAPEUTISCHE VERWENDUNG

1H-PYRIDO[3,4-b]INDOLE-4-CARBOXAMIDE DERIVATIVES, PREPARATION AND APPLICATION THEREOF IN THERAPEUTICS

(84) Etats contractants désignés:
**AT BE CH DE DK ES FI FR GB GR IE IT LI LU NL PT SE**
Etats d'extension désignés:
**AL LT LV RO SI**

(30) Priorité: **08.10.1996 FR 9612229**

(43) Date de publication de la demande: **11.08.1999 Bulletin 1999/32**

(73) Titulaire: **SANOFI-SYNTHELABO**
**75013 Paris (FR)**

(72) Inventeurs:
- **EVANNO, Yannick**
  **F-78830 Bullion (FR)**
- **SEVRIN, Mireille**
  **F-75014 Paris (FR)**
- **MALOIZEL, Christian**
  **F-92190 Meudon (FR)**
- **LEGALLOUDEC, Odette**
  **F-91150 Morigny (FR)**
- **GEORGE, Pascal**
  **F-78730 Saint Arnoult en Yvelines (FR)**

(74) Mandataire: **Ludwig, Jacques et al**
**Sanofi-Synthélabo**
**Service Brevets**
**174, avenue de France**
**75013 Paris (FR)**

(56) Documents cités:
**WO-A-96/08490**

- **C. HERDEIS ET AL.: "Synthesis and serotonin-receptor activity of substituted 1-oxo-1,2,3,4-tetrahydro-beta-carbolines" ZEITSCHRIFT FUR NATURFORSCHUNG, TEIL B: ANORGANISCHE CHEMIE,ORGANISCHE CHEMIE, vol. 42B, 1987, TUBINGEN,DE, pages 785-790, XP000674637**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet des dérivés de 1*H*-pyrido[3,4-*b*]indole-4-carboxamide, leur préparation et leur application en thérapeutique.

Les composés de l'invention répondent à la formule générale (I)

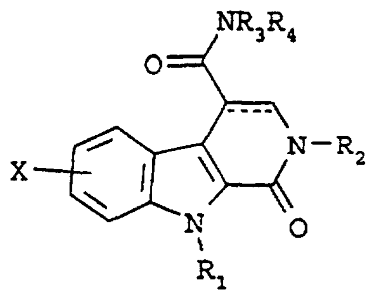

(I)

dans laquelle

X représente un atome d'hydrogène ou d'halogène ou un groupe $(C_1-C_3)$alkyle, $(C_1-C_3)$alcoxy, trifluorométhyle ou phénylméthoxy,
$R_1$ représente un atome d'hydrogène ou un groupe $(C_1-C_3)$-alkyle, cyclopropylméthyle ou phénylméthyle,
$R_2$ représente soit un groupe $(C_1-C_3)$alkyle éventuellement substitué par un groupe méthoxy, soit un groupe phényl-$(C_1-C_3)$alkyle éventuellement substitué sur le noyau phényle par un atome d'halogène ou un groupe méthyle ou méthoxy, soit un groupe cyclohexylméthyle, soit un groupe thiénylméthyle, soit un groupe pyridinylméthyle, soit un groupe phényle éventuellement substitué par un ou plusieurs atomes d'halogène ou un groupe $(C_1-C_3)$alkyle ou $(C_1-C_3)$alcoxy, soit un groupe pyridinyle, soit un groupe 5-méthyl-1,2-oxazolyle, soit un groupe 5-méthyl-1,3,4-thiadiazolyle, soit un groupe naphtalényle,
$R_3$ et $R_4$, indépendamment l'un de l'autre, représentent chacun un atome d'hydrogène, un groupe $(C_1-C_3)$alkyle, un groupe 2-méthoxyéthyle, un groupe hydroxy$(C_2-C_4)$alkyle, un groupe carboxy$(C_1-C_3)$alkyle, un groupe $(C_1-C_3)$ alcoxycarbonyl $(C_1-C_3)$-alkyle ou un groupe phényl$(C_1-C_3)$alkyle, ou bien forment ensemble, avec l'atome d'azote qui les porte, soit un groupe pyrrolidinyle éventuellement substitué par un groupe hydroxy, éthoxy, méthoxycarbonyle ou méthoxyméthyle, soit un groupe pipéridinyle, soit un groupe morpholinyle, soit un groupe 4-méthylpipérazinyle, soit un groupe azétidinyle, soit un groupe thiazolidinyle, et
la liaison entre les atomes de carbone en positions 3 et 4 est simple ou double.

Selon la nature de cette liaison, un composé selon l'invention peut éventuellement exister sous forme d'isomère optique pur ou d'un mélange de tels isomères.

Les composés préférés sont ceux dans la formule générale desquels X est en position 6 et représente un atome de fluor, $R_1$ représente un groupe méthyle, $R_2$ représente un groupe phényle, $R_3$ représente un groupe méthyle et $R_4$ représente un groupe éthyle ou bien $R_3$ et $R_4$ forment, avec l'atome d'azote qui les porte, un cycle pyrrolidinyle.

Les composés de formule générale (I) peuvent être préparés par des procédés illustrés par les schémas qui suivent.

Selon le schéma 1, le composé de départ répond à la formule générale (II), dans laquelle X et $R_1$ sont tels que définis ci-dessus et R représente un groupe $(C_1-C_3)$ alkyle ; lorsque $R_1$ représente l'hydrogène, on peut, si on le désire, effectuer une alkylation pour aboutir à un composé de formule générale (II) dans laquelle $R_1$ représente un groupe $(C_1-C_3)$alkyle.
On fait donc réagir le composé de formule générale (II) avec le pyruvate d'éthyle de formule (III), en milieu acide, par exemple en présence d'acide chlorhydrique gazeux dans l'éthanol, ou en présence d'acide sulfurique ou d'éthérate de trifluorure de bore dans l'acide acétique, entre la température ambiante et la température du reflux, pour obtenir le diester de formule générale (IV).
On traite ensuite ce dernier dans l'éthanol à la température du reflux avec une amine de formule générale $R_2NH_2$, dans laquelle $R_2$ est tel que défini ci-dessus. On obtient un ester de formule générale (V), qu'on transforme en l'acide correspondant, de formule générale (VI) par hydrolyse en milieu basique.
On transforme ensuite cet acide en amide primaire, secondaire ou tertiaire de formule générale (I') par action d'une

Schéma 1

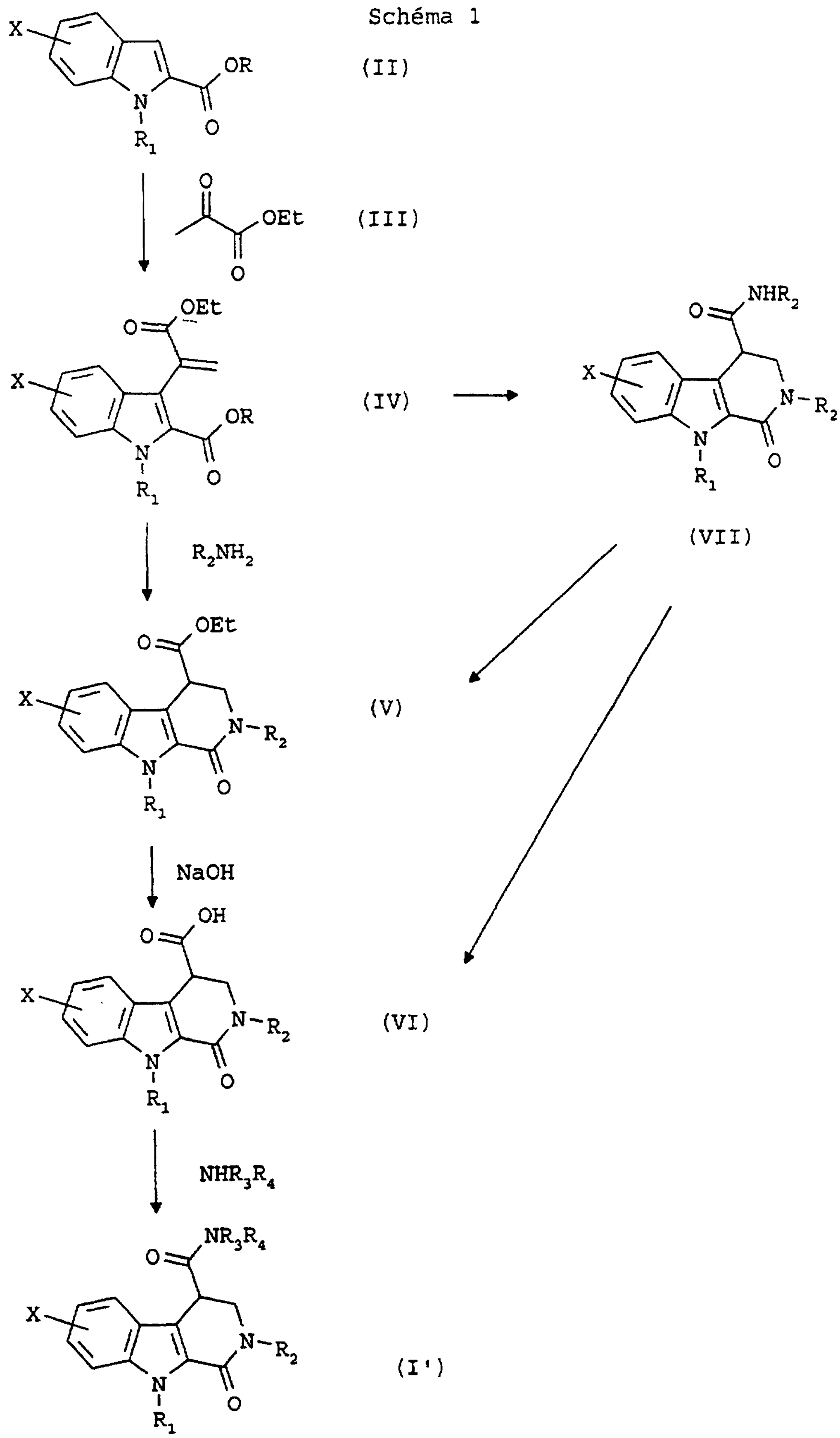

amine de formule générale $HNR_3R_4$, dans laquelle $R_3$ et $R_4$ sont tels que définis ci-dessus, soit en passant par l'imidazolide obtenu par action du *N,N'*-carbonyldiimidazole, soit en passant par le chlorure d'acide.
Dans le composé de formule générale (I') ainsi obtenu, la liaison entre les positions 3 et 4 est une liaison simple. Si l'on désire préparer un composé dans lequel cette liaison est une liaison double, on soumet un composé de formule générale (I') à une oxydation au moyen de 2,3-dichloro-5,6-dicyanocyclohexa-2,5-diène-1,4-dione ou de 3,4,5,6-tétrachlorocyclohexa-3,5-diène-1,2-dione dans un solvant tel que le toluène ou le dichlorométhane, entre la température ambiante et la température de reflux, pour obtenir le composé correspondant, dans la structure duquel la liaison entre les atomes de carbone en positions 3 et 4 est double, et répondant, respectivement, à la formule générale (I") :

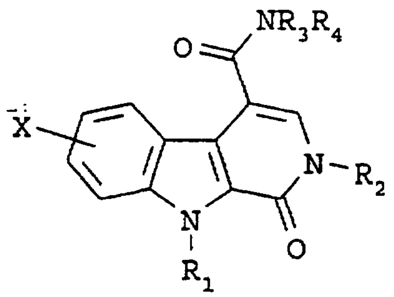

(I")

Enfin, et s'il y a lieu, on peut préparer les énantiomères à partir des racémates selon toutes méthodes connues ; ainsi, par exemple, on peut faire réagir un acide de formule générale (VI) avec une amine chirale optiquement pure telle que l'α-méthylbenzylamine, séparer les diastéréoisomères par cristallisation fractionnée, pour parvenir à un acide optiquement pur, puis aux esters et amides qui en dérivent.

Dans le cas d'un acide de formule générale (VI) optiquement pur, la réaction de couplage non racémisante peut être réalisée par toute méthode connue, par exemple avec utilisation d'hexafluorophosphate de (benzotriazol-1-yloxy)tris(pyrrolidin-1-yl)phosphonium.

Dans le cas où $R_2$ est un noyau aromatique, on peut, si on le désire, effectuer la transformation du diester (IV) en amide (VII) en chauffant le milieu réactionnel à une température de 100 à 200°C, dans un solvant inerte ou sans solvant, par exemple à reflux de l'amine correspondante de formule générale $R_2NH_2$. On peut ensuite tranformer le composé de formule générale (VII) soit en ester de formule générale (V), dans l'éthanol à reflux, en milieu acide, par exemple en présence d'acide chlorhydrique concentré, soit en acide de formule générale (VI) par hydrolyse en milieu basique.

Les composés de départ de formule générale (II), principalement avec $R_1$=H, sont décrits dans la littérature ; le pyruvate de formule (III) est disponible dans le commerce.

Selon le schéma 2 le composé de départ répond à la formule générale (VIII) dans laquelle X est tel que défini ci-dessus. On fait réagir ce composé avec l'acide 2-cétoglutarique puis on le traite dans un milieu alcoolique acide, par exemple dans l'éthanol saturé d'acide chlorhydrique gazeux, à la température de reflux pour obtenir le diester de formule générale (IX) dans laquelle R représente un groupe ($C_1$-$C_3$) alkyle. On effectue ensuite, si on le désire, une réaction d'alkylation de ce composé pour obtenir le composé de formule générale (X) dans laquelle $R_1$ représente un groupe ($C_1$-$C_3$) alkyle, puis on transforme ce dernier, dans un solvant protique, par exemple le *N,N*-diméthylformamide, en présence de diméthylacétaldiméthylformamide, à la température de reflux, pour obtenir le composé de formule (XI).
Si on le désire, on peut transformer directement le composé de formule générale (IX) en composé de formule générale (XI) dans laquelle $R_1$ représente un groupe méthyle dans les conditions précédemment décrites.
On traite ensuite le composé de formule générale (XI) par une amine de formule générale $H_2NR_2$, dans laquelle $R_2$ est tel que défini ci-dessus, dans un solvant protique, par exemple le *N,N*-diméthylformamide, en présence éventuellement d'un acide, par exemple l'acide 4-méthylbenzènesulfonique, à la température de reflux, pour obtenir l'ester de formule générale (V'). On transforme ce dernier en acide correspondant de formule générale (VI') par hydrolyse en milieu basique.

Enfin on transforme cet acide en amide primaire, secondaire ou tertiaire de formule générale (I") soit en passant par l'imidazolide obtenu par action du *N,N'*-carbonyldimidazole, soit en passant par le chlorure d'acide.

Schéma 2

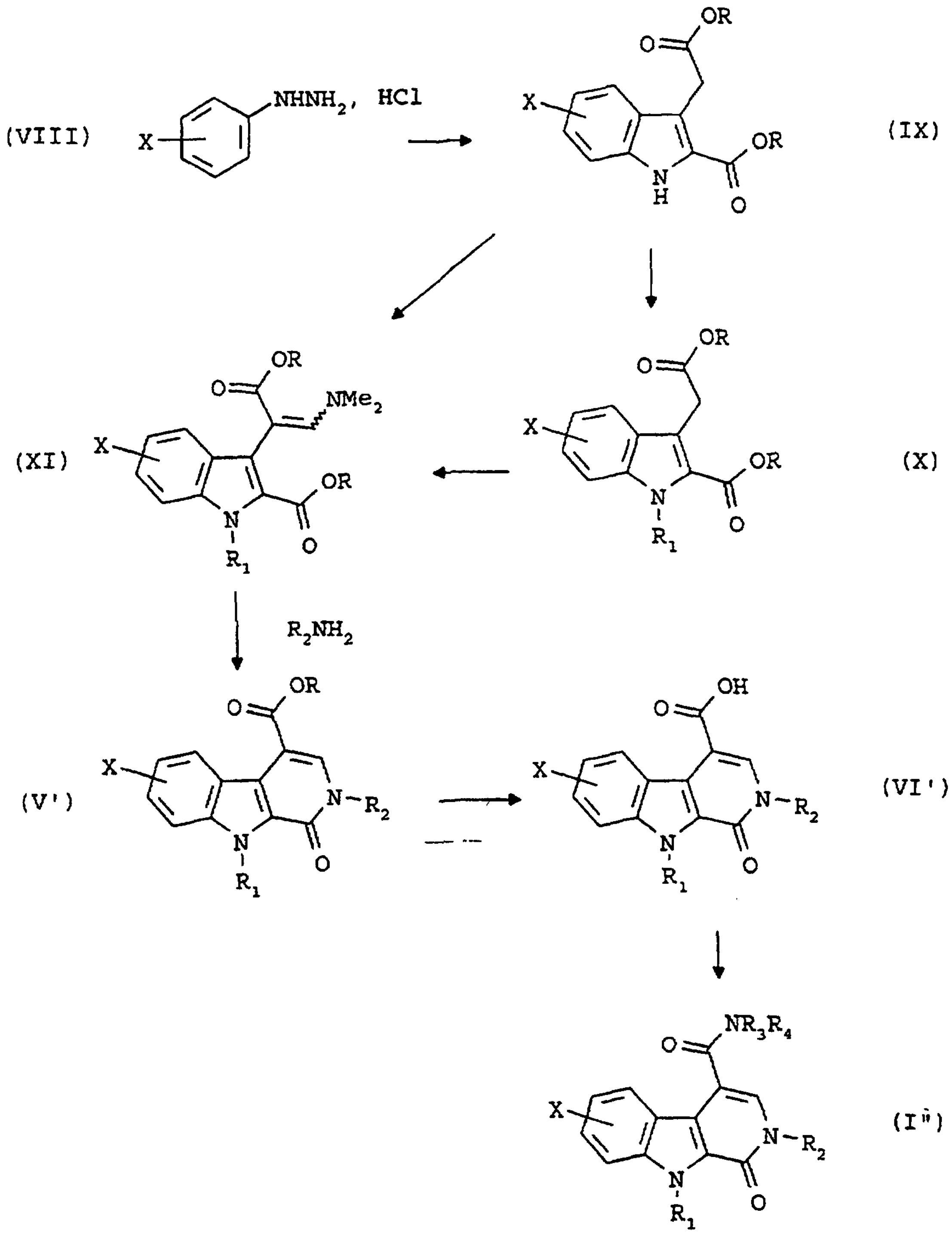


(VIII) (IX) (XI) (X) (V') (VI') (I")

Schéma 3

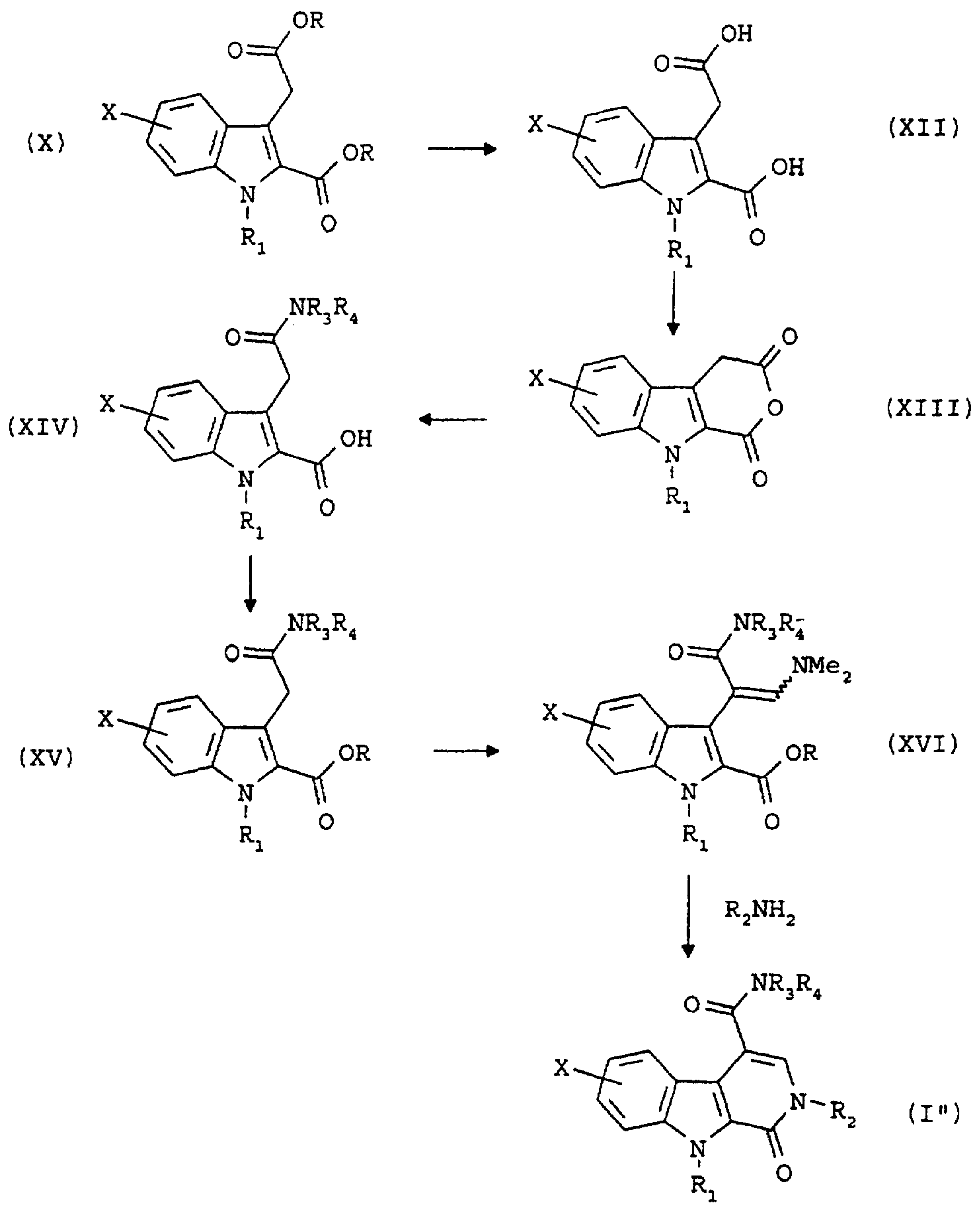


Les composés de départ de formule générale (VIII) sont disponibles dans le commerce. Certains composés de formules générales (IX) et (X) sont décrits dans la littérature.

Selon le schéma 3 on part du diester de formule générale (X), décrit à propos du procédé du schéma 2. On hydrolyse ce diester en milieu acide pour obtenir le diacide de formule générale (XII), que l'on transforme en anhydride, par exemple en utilisant le chlorure d'acétyle à la température de reflux, pour obtenir le composé de formule générale (XIII). On transforme ce dernier par action d'une amine de formule générale $HNR_3R_4$, dans laquelle $R_3$ et $R_4$ sont tels que définis ci-dessus, dans un solant chloré, par exemple le dichlorométhane, pour obtenir le composé de formule

générale(XIV), que l'on transforme en ester de formule générale (XV), puis on traite ce dernier dans un solvant protique, par exemple le *N,N*-diméthylformamide, en présence de diméthylacétaldiméthylformamide à la température de reflux, pour obtenir le composé de formule générale (XVI), et finalement on fait réagir ce dernier avec une amine de formule générale $R_2NH_2$, dans laquelle $R_2$ est tel que défini ci-dessus, dans un solvant protique, par exemple le *N,N*-diméthylformamide, en présence éventuellement d'un acide, par exemple l'acide 4-méthylbenzènesulfonique à la température de reflux pour obtenir le composé de formule générale (I'').
Enfin, si on le désire, on peut transformer un amide secondaire de formule générale (I''), dans laquelle $R_3$ ou $R_4$ représente l'hydrogène, en amide tertiaire par une réaction d'alkylation connue de l'homme du métier, au moyen d'un agent d'alkylation, par exemple un halogénure d'alkyle.
De même, on peut transformer un composé de formule générale (I') ou (I''), dans laquelle $R_1$ représente un atome d'hydrogène en un composé dans la formule duquel $R_1$ représente un groupe alkyle par une réaction d'alkylation de type connu. Des composés de structures chimiques analogues à celle des composés de l'invention sont décrits dans *CA* **83**(13) 114712c, *CA* **94**(9) 64698g et *CA* **96**(9) 68779y.

Certains composés de formules générales (XII), (XIII), (XIV) et (XV) sont décrits dans la littérature.
Un composé de formule générale (I) dans laquelle $R_3$ et/ou $R_4$ représentent un groupe hydroxy($C_2$-$C_4$)alkyle peut être obtenu par réaction de l'acide correspondant de formule générale (VI) ou (VI') avec un alcool protégé par un groupe protecteur classique, suivie d'une déprotection.
Un composé de formule générale (I) dans laquelle $R_3$ et/ou $R_4$ représentent un groupe carboxy($C_2$-$C_4$)alkyle peut être obtenu par hydrolyse d'un ester correspondant. Un composé de formule générale (I) dans laquelle X représente un groupe phénylméthoxy peut être obtenu en deux étapes, connues de l'homme du métier, à partir d'un composé de formule générale (I) dans laquelle X représente un groupe méthoxy.

Les exemples suivants illustrent en détail la préparation de quelques composés selon l'invention. Les microanalyses élémentaires et les spectres IR et RMN confirment les structures des composés obtenus.
Les numéros des composés indiqués entre parenthèses dans les titres correspondent à ceux du tableau donné plus loin.

<u>Exemple 1</u> (Composé N°20)

(±)-6-Fluoro-*N,N*,9-triméthyl-1-oxo-2-(phénylméthyl)-2,3,4,9-tétrahydro-1*H*-pyrido[3,4-*b*]indole-4-carboxamide.

1.1. 5-Fluoro-1-méthyl-1*H*-indole-2-carboxylate d'éthyle.

1.1.1. 5-Fluoro-1*H*-indole-2-carboxylate d'éthyle.

On ajoute, tout en refroidissant le mélange, 150 g (0,92 mole) de chlorhydrate de 4-fluorophénylhydrazine à une solution préalablement préparée de 23 g (1 mole) de sodium dans 1,5 l de méthanol, et on agite le mélange à température ambiante pendant 30 min.
On le concentre sous pression réduite, on reprend le résidu par du dichlorométhane et on sépare le chlorure de sodium par filtration. On évapore le solvant sous pression réduite, on dissout le résidu dans 830 ml d'éthanol contenant 4,4 ml d'acide acétique et 102 ml (0,91 mole) de pyruvate d'éthyle, et on chauffe au reflux pendant 2h.
On concentre le milieu réactionnel sous pression réduite, on . reprend le résidu avec de l'acétate d'éthyle, on lave la solution à l'eau, on la sèche sur sulfate de sodium et on évapore le solvant sous pression réduite. On obtient 181,6 g (0,83 mole) d'hydrazone.
On déshydrate 214 g (1,12 mole) d'acide 4-méthylbenzènesulfonique monohydraté en solution dans 2,5 1 de toluène en chauffant le milieu réactionnel pendant 2h au reflux dans un appareil de Dean-Stark. On ajoute, à froid, 181,6 g (0,83 mole) de l'hydrazone obtenue précédemment et on chauffe au reflux pendant 3h.
On refroidit le mélange, on ajoute de l'acétate d'éthyle et de l'eau, on sépare la phase organique, on la sèche et on évapore le solvant sous pression réduite. On recristallise le résidu dans du propan-2-ol et on purifie les eaux-mères par chromatographie sur colonne de gel de silice en éluant avec du dichlorométhane. On obtient 144 g (0,7 mole) de produit que l'on utilise tel quel dans l'étape suivante.

1.1.2. 5-Fluoro-1-méthyl-1*H*-indole-2-carboxylate d'éthyle.

On lave 11,7 g (0,39 mole) d'hydrure de sodium en suspension à 80% dans de l'huile avec de l'éther de pétrole puis on ajoute une solution de 62,1 g (0,3 mole) de 5-fluoro-1*H*-indole-2-carboxylate d'éthyle dans 600 ml de diméthylformamide. On agite pendant 2h à température ambiante, puis on ajoute 24,3 ml (0,39 mole) d'iodure de méthyle en solution dans 50 ml de diméthylformamide. On agite le mélange pendant 20h à température ambiante puis on le verse sur de l'eau glacée. On extrait le milieu réactionnel à l'acétate d'éthyle, on lave la phase organique et on la sèche

sur sulfate de sodium, on évapore le solvant sous pression réduite et on obtient 62,5 g (0,28 mole) de produit solide que l'on utilise tel quel dans l'étape suivante.

1.2. 2-(Ethoxycarbonyl)-5-fluoro-1-méthyl-α-méthylène-1*H*-indole-3-acétate d'éthyle.

On agite à température ambiante pendant 1h30 une solution de 10,5 g (48 mmoles) de 5-fluoro-1-méthyl-1*H*-indole-2-carboxylate d'éthyle, 18 g (155 mmoles) de pyruvate d'éthyle et 7,8 ml d'acide sulfurique concentré dans 100 ml d'acide acétique. On concentre le mélange sous pression réduite, on l'hydrolyse avec de l'eau glacée, on ajoute de l'ammoniaque jusqu'à pH alcalin et on extrait au dichlorométhane. On lave la phase organique à l'eau, on la sèche sur sulfate de sodium, on évapore le solvant sous pression réduite et on recristallise le résidu dans un mélange de pentane et d'éther diéthylique. On obtient 13 g (42 mmoles) de solide.
Point de fusion : 86-88°C.

1.3. (±)-6-Fluoro-9-méthyl-1-oxo-2-(phénylméthyl)-2,3,4,9-tétrahydro-1*H*-pyrido[3,4-*b*]indole-4-carboxylate d'éthyle.

On chauffe au reflux pendant 8h une solution de 7 g (23 mmoles) de 2-(éthoxycarbonyl)-5-fluoro-1-méthyl-α-méthylène-1*H*-indole-3-acétate d'éthyle et 15 ml (140 mmoles) de benzylamine dans 200 ml d'éthanol. On évapore le solvant sous pression réduite et on reprend le résidu par du dichlorométhane et de l'acide chlorhydrique 1N. On lave la phase organique à l'eau et on la sèche sur sulfate de sodium. On évapore le solvant sous pression réduite et on purifie le résidu par chromatographie sur colonne de gel de silice en éluant avec un mélange de dichlorométhane et d'acétate d'éthyle. On obtient 7 g (18 mmoles) de produit solide que l'on utilise tel quel dans l'étape suivante.

1.4. Acide (±)-6-fluoro-9-méthyl-1-oxo-2-(phénylméthyl)-2,3,4, 9-tétrahydro-1*H*-pyrido[3,4-*b*] indole-4-carboxylique.

On hydrolyse 6 g (16 mmoles) de (±)-6-fluoro-9-méthyl-1-oxo-2-(phénylméthyl)-2,3,4,9-tétrahydro-1*H*-pyrido [3,4-b]indole-4-carboxylate d'éthyle par 2,5 g de soude dans un mélange d'eau et d'éthanol, on concentre sous pression réduite, on ajoute de l'eau et de l'acide acétique, on extrait à l'acétate d'éthyle, on lave la phase organique à l'eau, on la sèche sur sulfate de sodium et on évapore le solvant sous pression réduite. On obtient 4 g (11 mmoles) de solide que l'on utilise tel quel dans l'étape suivante.
Point de fusion : 264-265°C.

1.5. (±) -6-Fluoro-*N,N*,9-triméthyl-1-oxo-2-(phénylméthyl)-2,3,4,9-tétrahydro-1*H*-pyrido[3,4-b]indole-4-carboxamide.

On chauffe à 40°C pendant 2h une solution de 4 g (11 mmoles) d'acide (±)-6-fluoro-9-méthyl-1-oxo-2- (phénylméthyl)-2,3,4,9-tétrahydro-1*H*-pyrido[3,4-*b*]indole-4-carboxylique et 2,2 g (13 mmoles) de 1,1'-carbonyldiimidazole dans 200 ml de tétrahydrofurane. On refroidit le milieu réactionnel et on ajoute de la diméthylamine liquéfiée en large excès et on laisse agiter pendant quelques heures.
On évapore le solvant sous pression réduite, on reprend le résidu par du dichlorométhane et de l'eau, on sépare la phase organique, on la lave à l'eau, on la sèche sur sulfate de sodium, on évapore le solvant sous pression réduite et on purifie le résidu par chomatographie sur colonne de gel de silice en éluant avec un mélange de dichlorométhane et de méthanol. On recristallise le produit dans l'acétate d'éthyle. On obtient 1,2 g (3 mmoles) de produit.
Point de fusion : 185-186°C.

<u>Exemple 2</u> (Composé N°59)

6-Fluoro-*N,N*,9-triméthyl-1-oxo-2-(phénylméthyl)-2,9-dihydro-1*H*-pyrido[3,4-*b*]indole-4-carboxamide.

On agite pendant 1h une solution de 2 g (5 mmoles) de (±)-6-fluoro-*N,N*,9-triméthyl-1-oxo-2-(phénylméthyl) -2,3,4,9-tétra-hydro-1*H*-pyrido[3,4-*b*]indole-4-carboxamide et de 1,6 g (7 mmoles) de 2,3-dichloro-5,6-dicyano-1,4-benzoquinone dans 250 ml de dichlorométhane. On lave la phase organique et on la sèche sur sulfate de sodium. On évapore le solvant sous pression réduite et on purifie le résidu par chromatographie sur colonne de gel de silice en éluant avec un mélange de dichlorométhane et d'acétate d'éthyle. On cristallise le produit dans l'éther diéthylique. On obtient 1 g (2,6 mmoles) de produit.
Point de fusion : 192-193°C.

Exemple 3 (Composé N°36)

6-Chloro-2- (2-méthoxyéthyl)-*N,N*,9-triméthyl-1-oxo-2,3,4,9-tétrahydro-1*H*-pyrido[3,4-*b*]indole-4-carboxamide.

3.1. 5-Chloro-1-méthyl-1*H*-indole-2-carboxylate d'éthyle.

On opère comme dans l'exemple 1.1.2 à partir de 8,95 g (40 mmoles) de 5-chloro-1*H*-indole-2-carboxylate d'éthyle, 1,6 g (52 mmoles) d'hydrure de sodium en suspension à 80% dans l'huile et 11,35 g (80 mmoles) d'iodure de méthyle. On obtient 9,5 g (40 mmoles) de produit solide que l'on utilise tel quel dans l'étape suivante.

3.2. 5-Chloro-2-(éthoxycarbonyl)-1-méthyl-α-méthylène-1*H*-indole-3-acétate d'éthyle.

On chauffe au reflux pendant 4h une solution saturée en acide chlorhydrique et contenant 9,5 g (40 mmoles) de 5-chloro-1-méthyl-1*H*-indole-2-carboxylate d'éthyle et 8,8 ml (80 mmoles) de pyruvate d'éthyle. On évapore le solvant sous pression réduite, on reprend le résidu à l'acétate d'éthyle que l'on lave jusqu'à neutralité, on sèche la phase organique sur sulfate de sodium et on évapore le solvant sous pression réduite. On purifie le produit par chromatographie sur colonne de gel de silice en éluant avec du dichlorométhane. On obtient 9,6 g (32 mmoles) de produit solide que l'on utilise tel quel dans l'étape suivante.

3.3. (±)-6-Chloro-2-(2-méthoxyéthyl)-9-méthyl-1-oxo-2,3,4,9-tétrahydro-1*H*-pyrido[3,4-*b*]indole-4-carboxylate d'éthyle.

On chauffe au reflux pendant 3h une solution de 9,5 g (31 mmoles) de 5-chloro-2-(éthoxycarbonyl)-1-méthyl-α-méthylène-1*H*-indole-3-acétate d'éthyle et 8,1 g (93 mmoles) de 2-méthoxyéthylamine dans 20 ml d'éthanol. On évapore le solvant sous pression réduite, on reprend le résidu par de l'acétate d'éthyle que l'on lave à l'eau et sèche sur sulfate de sodium. On obtient 9,7 g (27 mmoles) de produit solide que l'on utilise tel quel dans l'étape suivante:

3.4. Acide (±)-6-chloro-2-(2-méthoxyéthyl)-9-méthyl-1-oxo-2,3,4,9-tétrahydro-1*H*-pyrido[3,4-*b*]indole-4-carboxylique.

On hydrolyse 9,6 g (26 mmoles) de (±)-6-chloro-2-(2-méthoxyéthyl)-9-méthyl-1-oxo-2,3,4,9-tétrahydro-1*H*-pyrido[3,4-*b*]-indole-4-carboxylate d'éthyle par une solution de 3,1 g (80 moles) de soude dans 260 ml d'éthanol et 50 ml d'eau. On concentre le milieu réactionnel, on le reprend par de l'eau, on lave la phase aqueuse par de l'acétate d'éthyle et on acidifie jusqu'à pH=1 par de l'acide chlorhydrique concentré. On extrait à l'acétate d'éthyle. On lave à l'eau la phase organique et on la sèche sur sulfate de sodium. On évapore le solvant sous pression réduite et on obtient 8,3 g (26 mmoles) de produit solide que l'on utilise tel quel dans l'étape suivante.

3.5. (±)-6-Chloro-2-(2-méthoxyéthyl)-*N,N*,9-triméthyl-1-oxo-2,3,4,9-tétrahydro-1*H*-pyrido[3,4-*b*]indole-4-carboxamide.

On opère comme dans l'exemple 1.5 à partir de 8,3 g (26 mmoles) d'acide (±)-6-chloro-2-(2-méthoxyéthyl)-9-méthyl-1-oxo-2,3,4,9-tétrahydro-1*H*-pyrido[3,4-*b*]indole-4-carboxylique et de diméthylamine.-On isole 8,6 g de produit que l'on recristallise dans le propan-2-ol. On obtient 6,9 g (19 mmoles) de produit.
Point de fusion : 217-219°C.

Exemple 4 (Composé N°77)

(+)-6-fluoro-*N,N*,9-triméthyl-1-oxo-2-(phénylméthyl)-2,3,4,9-tétrahydro-1*H*-pyrido[3,4-*b*]indole-4-carboxamide.

4.1. Acide (+)-6-fluoro-9-méthyl-1-oxo-2-(phénylméthyl)-2,3,4,9-tétrahydro-1*H*-pyrido[3,4-*b*]indole-4-carboxylique.

On agite une solution de 20,5 g (58 mmoles) d'acide (±)-6-fluoro-9-méthyl-1-oxo-2-(phénylméthyl) -2,3,4,9-tétrahydro-1*H*-pyrido[3,4-*b*]indole-4-carboxylique et de 7,5 ml (58 mmoles) de (*R*)-(+)-α-méthylbenzylamine dans 1000 ml de méthanol. On concentre le mélange sous pression réduite, on reprend le résidu avec 50 ml d'acétate d'éthyle et 400 ml d'éther diéthylique, on sépare le précipité par filtration et on le recristallise trois fois dans le propan-2-ol. On isole 7,3 g (15 mmoles) de sel diastéréoisomère que l'on redissout dans 100 ml de méthanol, on ajoute 16 ml d'acide chlorhydrique 1N et 200 ml d'eau, on sépare le précipité par filtration et on le sèche sous pression réduite et à température ambiante. On obtient 5,1 g (15 mmoles) d'acide dextrogyre.
Point de fusion : 264-269°C.

$[\alpha]_D^{20}$ = +41,6° (c = 0,5 ; $CH_3OH$) ee > 99% (HPLC).

4.2. (+)-6-fluoro-*N*,*N*,9-triméthyl-1-oxo-2-(phénylméthyl)-2,3,4,9-tétrahydro-1*H*-pyrido[3,4-*b*]indole-4-carboxamide.

On refroidit à -30°C une solution de 0,5 g (1,3 mmole) d'acide (+)-6-fluoro-9-méthyl-1-oxo-2-(phénylméthyl)-2,3,4,9-tétrahydro-1*H*-pyrido[3,4-*b*]indole-4-carboxylique, 0,11 g (1,3 mmole) de chlorhydrate de diméthylamine préalablement séché sous pression réduite et 0,68 g (13 mmoles) d'hexafluorophosphate de (benzotriazol-1-yloxy)tris(pyrrolidino)phosphonium dans 10 ml de dichlorométhane préalablement passé sur colonne d'alumine, et on ajoute, goutte à goutte, 0,68 ml (3,9 mmoles) de *N,N*-di(1-méthyléthyl)éthylamine en solution dans 5 ml de dichlorométhane. On agite le mélange pendant 6h entre -30°C et -20°C, on l'hydrolyse avec 10 ml de solution aqueuse à 5% d'hydrogénosulfate de potassium, on extrait le mélange au dichlorométhane, on lave et sèche la phase organique, on évapore le solvant sous pression réduite, on purifie le résidu par chromatographie sur colonne de gel de silice en éluant avec un mélange de dichlorométhane et d'acétate d'éthyle et on recristallise le produit obtenu dans le propan-2-ol. On obtient 0,37 g (1 mmole) d'amide dextrogyre.
Point de fusion : 199-202°C.
$[\alpha]_D^{20}$ = +6,3° (c = 1 ; $CHCl_3$) ee > 94% (HPLC).

<u>Exemple 5</u> (Composé N°76)

(-)-6-Fluoro-*N*,*N*,9-triméthyl-1-oxo-2-(phénylméthyl)-2,3,4,9-tétrahydro-1*H*-pyrido[3,4-*b*]indole-4-carboxamide.

5.1. Acide (-)-6-fluoro-9-méthyl-1-oxo-2-(phénylméthyl)-2,3,4,9-tétrahydro-1*H*-pyrido[3,4-*b*]indole-4-carboxylique.

On évapore le solvant sous pression réduite des différentes eaux-mères de recristallisation de sel diastéréoisomère de l'exemple 4.1, on ajoute de l'eau et de l'acide chlorhydrique concentré, on sépare le précipité par filtration et on le sèche sous pression réduite et à température ambiante. On obtient 13,6 g (38 mmoles) d'acide lévogyre impur auxquels on ajoute 250 ml de méthanol et 4,97 ml (38 mmoles) de (*S*)-(-)-α-méthylbenzylamine.
On agite le mélange, on le concentre sous pression réduite, on recueille le précipité par filtration, on le recristallise trois fois dans le propan-2-ol. On obtient 7 g (15 mmoles) de sel diastéréoisomère que l'on redissout dans le minimum de méthanol, on ajoute 15 ml d'acide chlorhydrique 1N et on double le volume de la solution avec de l'eau. On sépare le précipité par filtration, on le lave à l'eau, on l'essore et on le sèche à température ambiante et sous pression réduite. On obtient 5 g (14 mmoles) d'acide lévogyre.
Point de fusion : 264-269°C.
$[\alpha]_D^{20}$ = -42,2° (c = 0,5 ; $CH_3OH$) ee > 99% (HPLC).

5.2. (-) -6-Fluoro-*N,N*,9-triméthyl-1-oxo-2-(phénylméthyl)-2,3,4,9-tétrahydro-1*H*-pyrido[3,4-*b*]indole-4-carboxamide.

En procédant comme décrit dans l'exemple 4.2, et à partir de l'acide (-)-6-fluoro-9-méthyl-1-oxo-2-(phénylméthyl)-2,3,4,9-tétrahydro-1*H*-pyrido[3,4-*b*]indole-4-carboxylique et de diméthylamine, on obtient, après recristallisation finale dans l'acétate d'éthyle, 0,3 g (0,8 mmole) d'amide lévogyre. Point de fusion : 204-205°C.
$[\alpha]_D^{20}$ = -7,5° (c = 1 ; $CHCl_3$) ee > 98% (HPLC).

<u>Exemple 6</u> (Composé N°101)

6-Fluoro-*N,N*,9-triméthyl-1-oxo-2-phényl-2, 9-dihydro-1*H*-pyrido[3,4-*b*]indole-4-carboxamide.

6.1. 2-(Ethoxycarbonyl)-5-fluoro-α-méthylène-1*H*-indole-3-acétate d'éthyle.

On agite une solution de 37,2 g (180 mmoles) de 5-fluoro-1*H*-indole-2-carboxylate d'éthyle, 25,8 g (222 mmoles) de pyruvate d'éthyle et 31 ml d'acide sulfurique concentré dans 400 ml d'acide acétique pendant 20 h.
On évapore le solvant sous pression réduite, on reprend le résidu avec de l'eau et de l'acétate d'éthyle, on sépare la phase organique, on la lave avec une solution d'ammoniaque diluée puis avec une solution aqueuse saturée de chlorure de sodium, on la sèche sur sulfate de sodium, on évapore le solvant sous pression réduite.
On obtient 37,1 g (122 mmoles) de produit solide qu'on utilise tel quel dans l'étape suivante.

6.2. Acide 6-fluoro-1-oxo-2-phényl-2,3,4,9-tétrahydro-1*H*-pyrido[3,4-*b*]indole-4-carboxylique.

On chauffe au reflux un mélange de 25 g (82 mmoles) de 2-(éthoxycarbonyl)-5-fluoro-α-méthylène-1*H*-indole-3-acétate d'éthyle et 31,8 g (342 mmoles) d'aniline pendant 17h.
On ajoute une solution d'acide chlorhydrique diluée et de l'acétate d'éthyle, on sépare la phase organique, on la lave à l'eau, on la sèche sur sulfate de sodium et on évapore le solvant sous pression réduite. On obtient 30 g de résidu qu'on hydrolyse par une solution de 43 ml de soude à 30% dans 400 ml d'éthanol au reflux, pendant 1h.
On concentre le mélange sous pression réduite, on ajoute de l'eau, on lave le mélange avec de l'acétate d'éthyle et du dichlorométhane, on acidifie la phase aqueuse avec de l'acide chlorhydrique concentré, on recueille le précipité par filtration et on le sèche sous pression réduite.
On obtient 18,5 g (57 mmoles) de composé solide qu'on utilise tel quel dans l'étape suivante.

6.3. 6-Fluoro-*N,N*,9-triméthyl-1-oxo-2-phényl-2,9-dihydro-1*H*-pyrido[3,4-*b*]indole-4-carboxamide.

On chauffe au reflux pendant 1h 5 g (15 mmoles) d'acide 6-fluoro-1-oxo-2-phényl-2,3,4,9-tétrahydro-1*H*-pyrido[3,4-*b*]indole-4-carboxylique dans 40 ml de chlorure de thionyle. On évapore le solvant sous pression réduite, on reprend le résidu avec du dichlorométhane, on ajoute un large excès de diméthylamine liquéfiée, on agite le mélange pendant quelques heures, on ajoute de l'eau, on sépare le précipité par filtration et on le sèche sous pression réduite.
On obtient 3,4 g (9 mmoles) de composé.
On en dissout 2,5 g dans 50 ml de diméthylsulfoxyde, on ajoute 0,6 g de potasse en poudre et 1,2 ml de iodométhane, et on agite le mélange pendant 5h à 50°C puis pendant 20h à température ambiante.
On ajoute de l'acide chlorhydrique dilué, on extrait à l'acétate d'éthyle, on sèche la phase organique sur sulfate de sodium, on évapore le solvant sous pression réduite et on purifie le résidu par chromatographie sur colonne de gel de silice en éluant avec un mélange de cyclohexane et d'acétate d'éthyle.
On obtient 2,2 g d'un mélange contenant le 6-fluoro-*N,N*,9-triméthyl-1-oxo-2-phényl-2,9-dihydro-1*H*-pyrido[3,4-*b*]indole-4-carboxamide et le 6-fluoro-*N,N*,9-triméthyl-1-oxo-2-phényl-2,3,4,9-tétrahydro-1*H*-pyrido[3,4-*b*]indole-4-carboxamide.
On agite ce mélange avec 1 g de 2,3-dichloro-5,6-dicyano-1,4-benzoquinone pendant 20h, on le lave avec une solution aqueuse saturée d'hydrogénocarbonate de sodium, on sépare la phase organique, on la sèche sur sulfate de sodium, on évapore le solvant sous pression réduite, on purifie le résidu par chromatographie sur colonne de gel de silice en éluant avec un mélange de cyclohexane et d'acétate d'éthyle et on recristallise le produit dans l'acétate d'éthyle.
On obtient 0,5 g (1,5 mmole) de composé.
Point de fusion : 195-197°C.

<u>Exemple 7</u> (Composé N°97)

*N,N*,9-Triméthyl-1-oxo-2-phényl-2,3,4,9-tétrahydro-1*H*-pyrido[3,4-*b*]indole-4-carboxamide.

7.1. 2-(Ethoxycarbonyl)-$\alpha$-méthylène-1*H*-indole-3-acétate d'éthyle

On porte à 60°c environ pendant 3h une solution d'éthanol saturée en acide chlorhydrique gazeux contenant 39,1 g (207 mmoles) de 1*H*-indole-2-carboxylate d'éthyle et 45,3 ml (410 mmoles) de pyruvate d'éthyle. On concentre sous pression réduite et on reprend le résidu à l'éther diéthylique. On lave la phase organique à l'eau et on la sèche sur sulfate de sodium. On évapore le solvant sous pression réduite et on fait cristalliser le résidu dans le cyclohexane. On obtient 45,4 g (158 mmoles) de produit solide que l'on utilise tel quel dans l'étape suivante.

7.2. 1-Oxo-*N*,2-diphényl-2,3,4,9-tétrahydro-1*H*-pyrido[3,4-*b*]indole-4-carboxamide.

On chauffe à reflux pendant 13h un mélange de 32 g (111 mmoles) de 2-(éthoxycarbonyl)-$\alpha$-méthylène-1*H*-indole-3-acétate d'éthyle et de 47,5 g (511 mmoles) d'aniline. On ajoute du dichlorométhane et on lave la phase organique par de l'acide chlorhydrique 1N. On la sèche sur sulfate de sodium et on évapore le solvant le solvant sous pression réduite. On obtient 38,8 g de produit impur que l'on utilise tel quel dans dans l'étape suivante.

7.3. 1-Oxo-2-phényl-2,3,4,9-tétrahydro-1*H*-pyrido[3,4-*b*]indole-4-carboxylate d'éthyle.

On chauffe à reflux une solution de 38,8 g de 1-oxo-*N*,2-diphényl-2,3,4,9-tétrahydro-1*H*-pyrido[3,4-*b*]indole-4-carboxamide impur dans un mélange d'éthanol, d'eau et d'acide chlorhydrique à 37% pendant 8h.
On neutralise le milieu réactionnel par de la soude concentrée et on extrait à l'acétate d'éthyle. On sèche la phase organique sur sulfate de sodium et on l'évapore sous pression réduite. On purifie le résidu par chromatographie sur colonne de gel de silice en éluant avec un mélange de dichlorométhane et d'acétate d'éthyle. On obtient 22,6 g (68 mmoles) de produit que l'on utilise tel quel dans l'étape suivante.

7.4. Acide 1-oxo-2-phényl-2,3,4,9-tétrahydro-1*H*-pyrido[3,4-*b*]indole-4-carboxylique.

On hydrolyse 22,6 g (68 mmoles) de 1-oxo-2-phényl-2,3,4,9-tétrahydro-1*H*-pyrido[3,4-*b*]indole-4-carboxylate d'éthyle par 200 ml de soude 1N dans 500 ml de méthanol. On acidifie le milieu réactionnel par de l'acide chlorhydrique 1N et on filtre le précipité. On le sèche sous pression réduite. On obtient 18,1 g (59 mmoles) de produit solide que l'on utilise tel quel dans dans l'étape suivante.

7.5. *N*,*N*-Diméthyl-1-oxo-2-phényl-2,3,4,9-tétrahydro-1*H*-pyrido[3,4-*b*]indole-4-carboxamide.

On chauffe à reflux pendant 4h une solution de 10 g (33 mmoles) d'acide 1-oxo-2-phényl-2,3,4,9-tétrahydro-1*H*-pyrido[3,4-*b*]indole-4-carboxylique dans 30ml de chlorure de thionyle. On évapore le solvant sous pression réduite, on reprend le résidu par du dichlorométhane et on ajoute de la diméthylamine liquéfiée en large excès. On agite plusieurs heures et on évapore le solvant sous pression réduite. On ajoute de l'eau et de l'acétate d'éthyle. On filtre le précipité et on le sèche sous pression réduite. On obtient 8,2 g de produit impur que l'on.utilise tel quel dans l'étape suivante.

7.6. *N,N*,9-Triméthyl-1-oxo-2-phényl-2,3,4,9-tétrahydro-1*H*-pyrido[3,4-*b*]indole-4-carboxamide.

On chauffe à 40°C pendant 30 min un mélange de 1,3 g (23 mmoles) d'hydroxyde de potassium en poudre et de 6 g de *N,N*-diméthyl-1-oxo-2-phényl-2,3,4,9-tétrahydro-1*H*-pyrido[3,4-*b*]indole-4-carboxamide impur dans 60 ml de diméthylsulfoxyde. On ajoute 2,5 ml (40 mmoles) d'iodométhane et on agite le milieu réactionnel pendant 5h à température ambiante.
On ajoute ensuite de l'eau et du dichlorométhane. On sèche la phase organique sur sulfate de sodium et on l'évapore sous pression réduite. On purifie le résidu par chromatographie sur colonne de gel de silice en éluant avec un mélange de dichlorométhane et d'acétate d'éthyle. On recristallise le produit dans l'acétate d'éthyle. On obtient 1,9 g (5,5 mmoles) de produit.
Point de fusion : 196-197°C.

Exemple 8 (Composé N°123)

*N*-Ethyl-6-fluoro-*N*,9-diméthyl-1-oxo-2-phényl-2,9-dihydro-1*H*-pyrido [3,4-*b*]indole-4-carboxamide.

8.1. 2-(Ethoxycarbonyl)-5-fluoro-1*H*-indole-3-acétate d'éthyle

On ajoute une solution de 7,4 g (185 mmoles) de soude dans 75 ml d'eau à une solution de 30 g (185 mmoles) de chlorhydrate de 4-fluorophénylhydrazine dans 300 ml d'eau, on agite pendant 15 min puis on ajoute une solution de 29 g (198 mmoles) d'acide cétoglutarique dans 60 ml d'eau. On agite à température ambiante pendant 3h, on extrait le milieu réactionnel par de l'acétate d'éthyle, on lave la phase organique à l'eau, on la sèche sur sulfate de sodium et on l'évapore sous pression réduite. On obtient 42 g (144 mmoles) de produit que l'on dissout dans 420 ml d'éthanol saturé d'acide chlorhydrique gazeux et on chauffe au reflux pendant 4h.
On concentre sous pression réduite, on reprend le résidu par de l'acétate d'éthyle, on lave la phase organique par de la soude normale puis à l'eau, on la sèche sur sulfate de magnésium et on l'évapore sous pression réduite. On obtient 42 g (143 mmoles) de produit solide que l'on utilise tel quel dans l'étape suivante.

8.2. 2-(Ethoxycarbonyl)-5-fluoro-1-méthyl-1*H*-indole-3-acétate d'éthyle.

On agite pendant 2h à température ambiante une solutiom de 7,36 g (184 mmoles) d'hydrure de sodium à 60% préalablement lavé à l'éther de pétrole et 45 g (153 mmoles) de 2-(éthoxycarbonyl)-5-fluoro-1*H*-indole-3-acétate d'éthyle dans 450 ml de *N*,*N*-diméthylformamide, puis on ajoute une solution de 19 ml (306 mmoles) d'iodométhane en solution dans 100 ml de *N,N*-diméthylformamide. Après 20h d'agitation on verse le mileu réactionnel sur de l'eau glaçée, on l'extrait par de l'éther diéthylique, on lave la phase organique à l'eau, on la sèche sur sulfate de magnésium et on évapore le solvant sous pression réduite. On obtient 44,3 g (144 mmoles) de produit que l'on utilise tel quel dans l'étape suivante.

8.3. $\alpha$-(Diméthylimino)-2-(éthoxycarbonyl)-5-fluoro-1-méthyl-1*H*-indole-3-acétate d'éthyle.

On chauffe au reflux pendant 50h une solution de 44,3 g (144 mmoles) de 2-(éthoxycarbonyl)-5-fluoro-1-méthyl-1*H*-indole-3-acétate d'éthyle et 57,4 ml de diméthylformamide diméthylacétal dans 450 ml de *N,N*-diméthylformamide. On évapore le solvant sous pression réduite et on reprend le résidu dans l'éther diéthylique. On élimine l'insoluble par filtration et on concentre le solvant sous pression réduite. On obtient 49,3 g (136 mmoles) de produit solide que l'on utilise tel quel dans l'étape suivante.

8.4. 6-Fluoro-9-méthyl-1-oxo-2-phényl-2,9-dihydro-1*H*-pyrido[3,4-*b*]indole-4-carboxylate d'éthyle.

On chauffe à reflux pendant 24h une solution de 16,3 g (45 mmoles) de $\alpha$-(diméthylimino)-2-(éthoxycarbonyl)-5-fluoro-1-méthyl-1*H*-indole-3-acétate d'éthyle, 4,64 ml (50 mmoles) d'aniline et 1,6 g (8 mmoles) d'acide 4-méthylbenzènesulfonique monohydraté dans 160 ml de *N,N*-diméthylformamide. On ajoute 3,3 g (31 mmoles) de carbonate de sodium par petites portions et on poursuit le reflux pendant 2h.
On refroidit la solution et on la verse sur de l'eau glaçée. On l'extrait par de l'acétate d'éthyle, on lave la phase organique à l'eau, on la sèche sur sulfate de magnésium et on l'évapore sous pression réduite. On purifie le résidu par chromatographie sur colonne de gel de silice en éluant avec un mélange de dichlorométhane et d'acétate d'éthyle. On obtient 10,9 g (30 mmoles) de produit que l'on utilise tel quel dans l'étape suivante.

8.5. Acide 6-Fluoro-9-méthyl-1-oxo-2-phényl-2,9-dihydro-1*H*-pyrido[3,4-*b*]indole-4-carboxylique.

On chauffe à reflux pendant 3h une solution de 22,8 g (65 mmoles) de 6-fluoro-9-méthyl-1-oxo-2-phényl-2,9-dihydro-1*H*-pyrido[3,4-*b*]indole-4-carboxylate d'éthyle et de 7,46 g (186 mmoles) de soude dans un mélange de 1 l d'éthanol et 100 ml d'eau. On concentre sous pression réduite, on reprend à l'eau et on lave la phase aqueuse par de l'acétate d'éthyle. On acidifie par de l'acide chlorhydrique concentré jusqu'à pH=1 et on filtre le produit que l'on rince plusieurs fois à l'eau. On le sèche sous pression réduite. On obtient 20,4 g (64 mmoles) de composé solide que l'on utilisé tel quel dans l'étape suivante.

8.6. 6-Fluoro-*N*,9-diméthyl-1-oxo-2-phényl-2,9-dihydro-1*H*-pyrido[3,4-*b*]indole-4-carboxamide.

On agite à 60°C pendant 4h une solution de 5 g (15,6 mmoles) d'acide 6-fluoro-9-méthyl-1-oxo-2-phényl-2,9-dihydro-1*H*-pyrido[3,4-*b*]indole-4-carboxylique et de 4,8 g (30 mmoles) de *N,N'*-carbonyldimidazole dans 100 ml de *N,N*-diméthylformamide. On ajoute à température ambiante un large excès de méthylamine liquéfié et on agite le milieu réactionnel pendant 20h. On verse la solution sur de l'eau glaçée, on recueille le précipité par filtration, on le lave avec une solution saturée d'hydrogénocarbonate de sodium, avec de l'eau puis avec de l'acétate d'éthyle, et on le sèche sous pression réduite. On isole 3,5 g de produit brut. On rassemble les filtrats et on les extrait par du dichlorométhane. On lave la phase organique avec une solution d'hydrogénocarbonate de sodium puis avec de l'eau, on la sèche sur sulfate de magnésium et on évapore le solvant sous pression réduite. On isole 1,5 g de produit supplémentaire. On rassemble les deux lots et on les purifie par chromatographie sur colonne de gel de silice en éluant avec un mélange de dichlorométhane et d'acétate d'éthyle. On obtient 4,7 g (13,5 moles) de produit solide.

8.7. *N*-Ethyl-6-fluoro-N,9-diméthyl-1-oxo-2-phényl-2,9-dihydro-1*H*-pyrido[3,4-*b*]indole-4-carboxamide.

On agite pendant 3h à 50°C une solution de 2,5 g (7,1 mmoles) de 6-fluoro-*N*,9-diméthyl-1-oxo-2-phényl-2,9-dihydro-1*H*-pyrido[3,4-*b*]indole-4-carboxamide et de 0,36 g (9 mmoles) d'hydrure de sodium à 60% préalablement lavé à l'éther de pétrole. On ajoute 1,67 ml (21 mmoles) d'iodoéthane, on maintient l'agitation pendant 20h, on verse la solution sur de l'eau glaçée et on l'extrait par de l'acétate d'éthyle. On lave la phase organique à l'eau, on la sèche sur sulfate de magnésium et on l'évapore sous pression réduite. On purifie le résidu par chromatographie sur silice en éluant avec un mélange de dichlorométhane et d'acétate d'éthyle. On recristallise le produit dans le propan-2-ol.
On obtient 2,3g de solide.
Pont de fusion : 181-182°C.

Exemple 9 (Composé N°98)

6-Fluoro-9-méthyl-2-phényl-4-(pyrrolidin-1-yl-carbonyl)-2,9-dihydro-1*H*-pyrido[3,4-*b*]indol-1-one.

On agite pendant 4h à 60°C une solution de 3,2 g (10 mmoles) d'acide 6-fluoro-9-méthyl-1-oxo-2-phényl-2,9-dihydro-1*H*-pyrido[3,4-*b*]indole-4-carboxylique et de 3,2 g (20 mmoles) de *N*,*N'*-carbonyldiimidazole dans 65 ml de *N*,*N*-diméthylformamide. On ajoute à température ambiante 2,5 ml (30 mmoles) de pyrrolidine et on agite le milieu réactionnel pendant 20h.
On verse la solution sur de l'eau glaçée et on l'extrait par de l'acétate d'éthyle. On lave la phase organique à l'eau, on la sèche sur sulfate de magnésium et on évapore le solvant sous pression réduite. On purifie le résidu par chromatographie sur colonne de gel de silice en éluant avec un mélange de dichlorométhane et d'acétate d'éthyle. On recristallise le produit dans le propan-2-ol.
On obtient 3g (7,7 mmoles) de solide.
Point de fusion : 203-205°C

Exemple 10 (Composé N°122)

6-Fluoro-*N*,*N*,9-Triméthyl-1-oxo-2-pyridin-2-yl-2,9-dihydro-1*H*-pyrido[3,4-*b*]indole-4-carboxamide.

10.1. 6-Fluoro-9-méthyl-1-oxo-2-pyridin-2-yl-2,9-dihydro-1*H*-pyrido[3,4-*b*]indole-4-carboxylate de méthyle.

On chauffe à 180°C pendant 30 min un mélange de 4,1 g (12,2 mmoles) de α-(diméthylimino)-5-fluoro-2-(méthoxycarbonyl)-1-méthyl-1*H*-indole-3-acétate de méthyle et de 1,73 g (18,4 mmoles) de 2-aminopyridine. On ajoute 7ml de *N*,*N*-diméthylformamide et on poursuit le chauffage pendant 4h.
On refroidit le milieu réactionnel, on le verse sur un mélange d'eau et d'acétate d'éthyle, on recueille l'insoluble par filtration et on le sèche sous pression réduite.
On obtient 1,8 g (5,1 mmoles) de solide que l'on utilise tel quel dans l'étape suivante.

10.2. Acide 6-fluoro-9-méthyl-1-oxo-2-pyridin-2-yl-2,9-dihydro-1*H*-pyrido[3,4-*b*]indole-4-carboxylique.

On chauffe à reflux pendant 4h une solution de 3,3 g (9,4 mmoles) de 6-fluoro-9-méthyl-1-oxo-2-pyridin-2-yl-2,9-dihydro-1*H*-pyrido[3,4-*b*]indole-4-carboxylate de méthyle dans un mélange de 100 ml d'éthanol et de 28 ml de soude 1N. On concentre sous pression réduite, on ajoute de l'eau, on acidifie par de l'acide chlorhydrique concentré et on recueille le précipité par filtration. On le lave à l'eau et on le sèche sous pression réduite. On obtient 2,8 g (8,3 mmoles) de produit solide que l'on utilise tel quel dans l'étape suivante.

10.3. 6-Fluoro-*N,N*,9-triméthyl-1-oxo-2-pyridin-2-yl-2,9-dihydro-1*H*-pyrido[3,4-*b*]indole-4-carboxamide.

On agite pendant 4h une solution de 2,5 g (7,4 mmoles) d'acide 6-fluoro-9-méthyl-1-oxo-2-pyridin-2-yl-2,9-dihydro-1*H*-pyrido[3,4-*b*]indole-4-carboxylique et de 2,4 g (14,8 mmoles) de *N*,*N'*-carbonyldiimidazole dans 65 ml de *N*,*N*-diméthylformamide. On refroidit le milieu réactionnel et on ajoute un large excès de diméthylamine liquéfiée. On agite pendant 48h à température ambiante, on verse dans l'eau et on extrait à l'acétate d'éthyle. On lave la phase organique à l'eau, on la sèche sur sulfate de magnésium et on évapore le solvant sous pression réduite. On purifie le résidu par chromatographie sur colonne de gel de silice en éluant avec un mélange de dichlorométhane et d'acétate d'éthyle. On recristallise le produit dans l'acétate d'éthyle.
On obtient 1,6 g (4,4 mmoles) de solide.
Point de fusion : 220-221°C

Exemple 11 (Composé N°125)

6-Fluoro-9-méthyl-2-(5-méthyl-1,3,4-thiadiazol-2-yl)-4-(pyrrolidin-1-yl-carbonyl)-2,9-dihydro-1*H*-pyrido[3,4-*b*]indol-1-one.

11.1. Acide 2-carboxy-5-fluoro-1*H*-indole-3-acétique.

On chauffe à reflux une solution de 26,5 g (103 mmoles) de 2-(éthoxycarbonyl)-5-fluoro-1*H*-indole-3-acétate d'éthyle et 24 g de soude dans un mélange de 530 ml d'éthanol et de 100 ml d'eau. On concentre sous pression réduite, on ajoute de l'eau, on lave la phase aqueuse par de l'acétate d'éthyle, on acidifie par de l'acide chlorhydrique concentré. On filtre le précipité, on le rince par de l'eau et on le sèche sous pression réduite. On obtient 23,4 g (98,7 mmoles) de produit que l'on utilise tel quel dans l'étape suivante.

11.2. 6-Fluoro-1,3,4,9-tétrahydropyrano[3,4-*b*]indole-1,3-dione.

On chauffe à reflux pendant 5h une solution de 4,7 g (19,8 mmoles) d'acide 2-carboxy-5-fluoro-1*H*-indole-3-acétique dans 94 ml de chlorure d'acétyle. On concentre sous pression réduite, on ajoute du toluène et on évapore les solvants sous pression réduite. On obtient 4,5 g de produit solide que l'on utilise tel quel dans l'étape suivante.

11.3. Acide 5-fluoro-3-(2-oxo-2-pyrrolidin-1-yléthyl)-1*H*-indole-2-carboxylique.

On agite pendant 24h à température ambiante une solution de 4,4 g (20 mmoles) de 6-fluoro-1,3,4,9-tétrahydropyrano[3,4-*b*]indole-1,3-dione et de 8,3 ml (100 mmoles) de pyrrolidine dans 100 ml de dichlorométhane. On concentre sous pression réduite, on ajoute de l'eau et on lave la phase aqueuse par de l'acétate d'éthyle. On acidifie par de l'acide chlorhydrique concentré et on ajoute de l'acétate d'éthyle. On recueille le précipité par filtration, on le lave à l'eau et on le sèche sous pression réduite. On obtient 5 g (17,2 mmoles) de produit solide que l'on utilise tel quel dans l'etape suivante.

11.4. 5-fluoro-3-(2-oxo-2-pyrrolidin-1-yléthyl)-1*H*-indole-2-carboxylate de méthyle.

On ajoute, goutte à goutte, 3,8 ml (51 mmoles) de chlorure de thionyle à une solution de 5 g (17,2 mmoles) d'acide 5-fluoro-3-(2-oxo-2-pyrrolidin-1-yléthyl)-1*H*-indole-2-carboxylique dans 50 ml de méthanol refroidi sur un bain de glace, puis on chauffe le mélange à reflux pendant 3h.
On concentre sous pression réduite, on ajoute de l'eau et du dichlorométhane, on lave la phase organique à l'eau, on la sèche sur sulfate de magnésium et on l'évapore sous pression réduite. On obtient 4,5 g (14 mmoles) de produit que l'on utilise tel quel dans l'étape suivante.

11.5. 3-(1-Diméthylimino- 2-oxo-2-pyrrolidin-1-yléthyl)-5-fluoro-1-méthyl-1*H*-indole-2-carboxylate de méthyle.

On chauffe à reflux pendant 30h une solution de 3,4 g de 5-fluoro-3-(2-oxo-2-pyrrolidin-1-yléthyl)-1*H*-indole-2-carboxylate de méthyle et 4,66 ml (35 mmoles) de diméthylformamide diméthylacétal dans 34 ml de *N*,*N*-diméthylformamide. On concentre sous pression réduite, on ajoute du xylène et on évapore les solvants sous pression réduite. On obtient 4 g de résidu contenant environ 50% du produit souhaité (d'après le spectre de résonance magnétique du proton) et que l'on utilise tel que dans l'étape suivante.

11.6. 6-Fluoro-9-méthyl-2-(5-méthyl-1,3,4-thiadiazol-2-yl)-4-(pyrrolidin-1-yl-carbonyl)-2,9-dihydro-1*H*-pyrido[3,4-*b*]indol-1-one.

On agite pendant 15 min une solution de 4 g du résidu obtenu dans l'étape précédente et 1,04 g (5,5 mmoles) d'acide 4-méthylbenzènesulfonique monohydraté dans 40 ml de *N*,*N*-diméthylformamide. On ajoute 0,65 g (6,4 mmoles) de 2-amino-5-méthyl-1,3,4-thiadiazole et on chauffe à reflux pendant 24h.
On verse le mélange dans de l'eau et de l'acétate d'éthyle. On recueille le précipité par filtration, on le lave à l'eau, on le sèche sous pression réduite et on le recristallise dans le *N,N*-diméthylformamide.
On obtient 1g (2,4 mmoles) de produit.
Point de fusion : 299-301°C.
Le tableau qui suit illustre les structures chimiques et les propriétés physiques de quelques composés selon l'invention.

Tableau

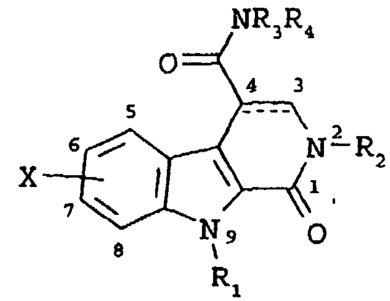

(I)

| N° | X | $R_1$ | $R_2$ | $NR_3R_4$ | 3~4 | F (°C) |
|---|---|---|---|---|---|---|
| 1 | H | Me | Pr | NHMe | / | 150-152 |
| 2 | H | Me | Pr | $NMe_2$ | / | 142-144 |
| 3 | H | Pr | Pr | $NMe_2$ | / | 125-126 |
| 4 | H | Me | $PhCH_2$ | $NEt_2$ | / | 107-109 |
| 5 | H | Me | $PhCH_2$ | $NMe_2$ | / | 162-163 |
| 6 | H | Pr | $PhCH_2$ | $NMe_2$ | / | 178-180 |
| 7 | H | Me | 4-MeO-$PhCH_2$ | $NMe_2$ | / | 149-150 |
| 8 | H | Me | 4-F-$PhCH_2$ | $NMe_2$ | / | 172-174 |
| 9 | H | Me | $PhCH_2CH_2$ | $NMe_2$ | / | 138-139 |
| 10 | H | Me | 4-Cl-$PhCH_2$ | $NMe_2$ | / | 168-170 |
| 11 | H | Et | $PhCH_2$ | $NMe_2$ | / | 172-174 |

| N° | X | $R_1$ | $R_2$ | $NR_3R_4$ | 3-4 | F (°C) |
|---|---|---|---|---|---|---|
| 12 | H | Me | 2-MeO-PhCH$_2$ | NMe$_2$ | / | 162-163 |
| 13 | H | Me | 3-MeO-PhCH$_2$ | NMe$_2$ | / | 139-140 |
| 14 | H | Me | 2-Me-PhCH$_2$ | NMe$_2$ | / | 154-156 |
| 15 | H | Me | 3-Me-PhCH$_2$ | NMe$_2$ | / | 137-139 |
| 16 | H | Et | 4-MeO-PhCH$_2$ | NMe$_2$ | / | 149-150 |
| 17 | H | Me | 4-Me-PhCH$_2$ | NMe$_2$ | / | 153-155 |
| 18 | H | Me | Ph(CH$_2$)$_3$ | NMe$_2$ | / | 118-120 |
| 19 | 5-F | Me | PhCH$_2$ | NMe$_2$ | / | 195-196 |
| 20 | 6-F | Me | PhCH$_2$ | NMe$_2$ | / | 185-186 |
| 21 | 7-F | Me | PhCH$_2$ | NMe$_2$ | / | 195-196 |
| 22 | 8-F | Me | PhCH$_2$ | NMe$_2$ | / | 188-190 |
| 23 | 6-MeO | Me | PhCH$_2$ | NMe$_2$ | / | 181-182 |
| 24 | 7-MeO | Me | PhCH$_2$ | NMe$_2$ | / | 208-210 |
| 25 | 6-Cl | Me | PhCH$_2$ | NMe$_2$ | / | 208-210 |
| 26 | 7-Cl | Me | PhCH$_2$ | NMe$_2$ | / | 209-210 |
| 27 | 8-Cl | Me | PhCH$_2$ | NMe$_2$ | / | 218-219 |
| 28 | 6-Me | Me | PhCH$_2$ | NMe$_2$ | / | 209-210 |

| N° | X | $R_1$ | $R_2$ | $NR_3R_4$ | 3-4 | F (°C) |
|---|---|---|---|---|---|---|
| 29 | 7-Me | Me | $PhCH_2$ | $NMe_2$ | / | 203-205 |
| 30 | 8-Me | Me | $PhCH_2$ | $NMe_2$ | / | 204-205 |
| 31 | 6-F | Me | $PhCH_2$ | $NEt_2$ | / | 120-121 |
| 32 | 6-F | Me | $PhCH_2$ | $NPr_2$ | / | 156-157 |
| 33 | 6-F | Et | 2-MeO-$PhCH_2$ | $NMe_2$ | / | 136-137 |
| 34 | 6-F | Me | $PhCH_2$ | NHMe | / | 205-206 |
| 35 | 6-F | Et | $PhCH_2$ | $NMe_2$ | / | 206-207 |
| 36 | 6-Cl | Me | $MeO(CH_2)_2$ | $NMe_2$ | / | 217-219 |
| 37 | 6-F | Me | $PhCH_2$ | NH2 | / | 259-260 |
| 38 | 6-F | Me | 2-Pyridyl-$CH_2$ | $NMe_2$ | / | 186-189 |
| 39 | 6-F | Me | 2-Thienyl-$CH_2$ | $NMe_2$ | / | 191-193 |
| 40 | 6-F | Me | 3-Pyridyl-$CH_2$ | $NMe_2$ | / | 200-202 |
| 41 | 6-F | Me | $C_6H_{11}CH_2$ | $NMe_2$ | / | 209-211 |
| 42 | 6-F | Me | $PhCH_2$ | Piperid | / | 205-206 |
| 43 | 6-F | Me | $PhCH_2$ | Pyrrolid | / | 228-229 |
| 44 | H | Pr | Pr | $NMe_2$ | // | 107-108 |
| 45 | H | Me | $PhCH_2$ | $NMe_2$ | // | 130-131 |

| N° | X | $R_1$ | $R_2$ | $NR_3R_4$ | 3-4 | F (°C) |
|---|---|---|---|---|---|---|
| 46 | H | Pr | $PhCH_2$ | $NMe_2$ | // | 160-162 |
| 47 | H | Me | 4-MeO-$PhCH_2$ | $NMe_2$ | // | 98-100 |
| 48 | H | Me | 4-F-$PhCH_2$ | $NMe_2$ | // | 105-106 |
| 49 | H | Me | 4-Cl-$PhCH_2$ | $NMe_2$ | // | 103-104 |
| 50 | H | Et | $PhCH_2$ | $NMe_2$ | // | 168-170 |
| 51 | H | Me | 2-MeO-$PhCH_2$ | $NMe_2$ | // | 171-173 |
| 52 | H | Me | 3-MeO-$PhCH_2$ | $NMe_2$ | // | 168-170 |
| 53 | H | Me | 3-Me-$PhCH_2$ | $NMe_2$ | // | 117-118 |
| 54 | H | Et | 4-MeO-$PhCH_2$ | $NMe_2$ | // | 127-128 |
| 55 | H | Me | 2-Me-$PhCH_2$ | $NMe_2$ | // | 157-158 |
| 56 | H | Me | 4-Me-$PhCH_2$ | $NMe_2$ | // | 98 -100 |
| 57 | H | Me | $Ph(CH_2)_3$ | $NMe_2$ | // | 107-109 |
| 58 | 5-F | Me | $PhCH_2$ | $NMe_2$ | // | 168-170 |
| 59 | 6-F | Me | $PhCH_2$ | $NMe_2$ | // | 192-193 |
| 60 | 7-F | Me | $PhCH_2$ | $NMe_2$ | // | 142-144 |
| 61 | 8-F | Me | $PhCH_2$ | $NMe_2$ | // | 190-192 |
| 62 | 6-MeO | Me | $PhCH_2$ | $NMe_2$ | // | 178-180 |

| N° | X | $R_1$ | $R_2$ | $NR_3R_4$ | 3-4 | F (°C) |
|---|---|---|---|---|---|---|
| 63 | 7-MeO | Me | $PhCH_2$ | $NMe_2$ | // | 155-156 |
| 64 | 6-Cl | Me | $PhCH_2$ | $NMe_2$ | // | 200-202 |
| 65 | 7-Cl | Me | $PhCH_2$ | $NMe_2$ | // | 183-184 |
| 66 | 6-Me | Me | $PhCH_2$ | $NMe_2$ | // | 158-160 |
| 67 | 7-Me | Me | $PhCH_2$ | $NMe_2$ | // | 154-156 |
| 68 | 8-Me | Me | $PhCH_2$ | $NMe_2$ | // | 168-169 |
| 69 | 6-F | Et | $PhCH_2$ | $NMe_2$ | // | 216-217 |
| 70 | 6-Cl | Me | $MeO-(CH_2)_2$ | $NMe_2$ | // | 179-180 |
| 71 | 6-F | Me | 2-Pyridyl-$CH_2$ | $NMe_2$ | // | 221-223 |
| 72 | 6-F | Me | 2-Thienyl-$CH_2$ | $NMe_2$ | // | 164-165 |
| 73 | 6-F | Me | $C_6H_{11}CH_2$ | $NMe_2$ | // | 170-172 |
| 74 | 6-F | Et | 2-MeO-$PhCH_2$ | $NMe_2$ | // | 199-200 |
| 75 | 6-F | Me | 3-Pyridyl-$CH_2$ | $NMe_2$ | // | 172- 175 |
| 76 | 6-F | Me | $PhCH_2$ | $NMe_2$ | / | 204-205 |
| 77 | 6-F | Me | $PhCH_2$ | $NMe_2$ | / | 199-202 |
| 78 | H | H | Pr | NHMe | / | 215-217 |
| 79 | H | H | Pr | $NMe_2$ | / | 202-204 |

| N° | X | $R_1$ | $R_2$ | $NR_3R_4$ | 3-4 | F (°C) |
|---|---|---|---|---|---|---|
| 80 | H | H | Pr | $NH_2$ | / | 220-222 |
| 81 | H | H | Pr | $NHCH_2Ph$ | / | 228-230 |
| 82 | H | H | Pr | $NMe_2$ | // | 244-247 |
| 83 | H | H | Ph | $NMe_2$ | // | 292-294 |
| 84 | 6-F | H | $PhCH_2$ | $NMe_2$ | // | 298-301 |
| 85 | 6-F | H | $PhCH_2$ | $NMe_2$ | / | 320-322 |
| 86 | 6-F | H | $PhCH_2$ | NHMe | / | 271-273 |
| 87 | 6-F | H | $PhCH_2$ | NHMe | // | >300 |
| 88 | H | H | $PhCH_2$ | $NMe_2$ | // | 268-269 |
| 89 | 6-F | H | Me | $NMe_2$ | // | >300 |
| 90 | 6-F | H | iPr | $NMe_2$ | // | 284-285 |
| 91 | 6-Cl | H | PhCH2 | $NMe_2$ | // | >300 |
| 92 | 6-MeO | H | PhCH2 | $NMe_2$ | // | >300 |
| 93 | 6-Me | H | PhCH2 | $NMe_2$ | // | 267-268 |
| 94 | 6-F | H | Ph | $NMe_2$ | // | 295-297 d |
| 95 | 6-F | H | Ph | $NMe_2$ | / | 300 d |
| 96 | 6-F | H | 2-Thienyl-$CH_2$ | $NMe_2$ | // | 318-320 |

| N° | X | $R_1$ | $R_2$ | $NR_3R_4$ | 3-4 | F (°C) |
|---|---|---|---|---|---|---|
| 97 | H | Me | Ph | $NMe_2$ | / | 196-197 |
| 98 | 6-F | Me | Ph | Pyrrolid | // | 203-205 |
| 99 | 6-F | Me | 3-Thienyl-$CH_2$ | $NMe_2$ | / | 196-197 |
| 100 | 6-F | Me | 3-Thienyl-$CH_2$ | $NMe_2$ | // | 172-174 |
| 101 | 6-F | Me | Ph | $NMe_2$ | // | 195-197 |
| 102 | 6-F | Me | Ph | $NMe_2$ | / | 213-215 |
| 103 | 6-F | Et | Ph | $NMe_2$ | // | 210-212 |
| 104 | 6-F | Me | 2-F-Ph | $NMe_2$ | // | 235-237 |
| 105 | 6-F | Me | 4-F-Ph | $NMe_2$ | // | 214-215 |
| 106 | 6-Cl | Me | Ph | $NMe_2$ | // | 195-196 |
| 107 | 6-F | Me | 4-Me-Ph | $NMe_2$ | // | 252-254 |
| 108 | 6-Me | Me | Ph | $NMe_2$ | // | 192-193 |
| 109 | H | Me | Ph | $NMe_2$ | // | 205-207 |
| 110 | 6-F | Me | Ph | $NHCH_2Ph$ | // | 243-244 |
| 111 | 6-F | Me | Ph | NHMe | // | 294-296 |
| 112 | 6-F | Me | 3-F-Ph | $NMe_2$ | // | 199-202 |
| 113 | 6-F | Me | 3-Cl-Ph | $NMe_2$ | // | 186-188 |

| N° | X | $R_1$ | $R_2$ | $NR_3R_4$ | 3-4 | F (°C) |
|---|---|---|---|---|---|---|
| 114 | 6-F | Me | 3-MeO-Ph | $NMe_2$ | // | 215 |
| 115 | 6-F | Me | Ph | Morph | // | 254-255 |
| 116 | 6-F | Me | Ph | 4-Me-piperaz | // | 224-226 |
| 117 | 6-F | Me | Ph | Piperid | // | 178-180 |
| 118 | 6-F | Me | Ph | $NEt_2$ | // | 164-165 |
| 119 | 6-F | Me | Ph | $N(Me)(CH_2)_2OMe$ | // | 172-174 |
| 120 | 6-F | Me | Ph | 3-HO-pyrrolid | // | 201-202 |
| 121 | 6-F | Me | Ph | 3-EtO-pyrrolid | // | 189-190 |
| 122 | 6-F | Me | 2-Pyridyl | $NMe_2$ | // | 220-221 |
| 123 | 6-F | Me | Ph | N(Me)Et | // | 181-182 |
| 124 | 6-F | Me | Ph | Pyrrolid | // | 203-205 |
| 125 | 6-F | Me | 5-Me-thiadiaz | Pyrrolid | // | 299-301 |
| 126 | 6-F | Et | Ph | Pyrrolid | // | 172-174 |
| 127 | 6-Cl | Me | Ph | Pyrrolid | // | 209-210 |
| 128 | 6-F | Me | 5-Me-oxazol | Pyrrolid | // | 248-250 |
| 129 | 6-F | Me | Ph | Azetid | // | 230-231 |
| 130 | 6-F | Me | Ph | N(Me)Pr | // | 172-173 |

| N° | X | $R_1$ | $R_2$ | $NR_3R_4$ | 3-4 | F (°C) |
|---|---|---|---|---|---|---|
| 131 | 6-F | $PhCH_2$ | Ph | $NMe_2$ | // | 201-203 |
| 132 | 6-F | Me | Ph | $N(Me)CH_2Ph$ | // | 148-149 |
| 133 | 6-F | $cPrCH_2$ | Ph | $NMe_2$ | // | 178-179 |
| 134 | 6-F | Me | 1-Napht | $NMe_2$ | // | 245-247 |
| 135 | 6-F | Me | 2-Napht | $NMe_2$ | // | 185-186 |
| 136 | 6-F | Me | Ph | (*S*)-2-$MeOCH_2$-pyrrolid | // | 96-107 |
| 137 | 6-F | Me | Ph | $N(Me)CH_2CH_2Ph$ | // | 174-176 |
| 138 | 6-F | Me | Ph | $N(Me)CH_2CO_2Et$ | // | 158-160 |
| 139 | 6-F | Me | Ph | 2-MeOCO-pyrrolid | // | 115-118 |
| 140 | 6-F | Me | Ph | $N(Me)CH_2CH_2Ph$ | // | 151-153 |
| 141 | 6-F | Me | Ph | Thiazolid | // | 187-188 |
| 142 | 6-MeO | Me | Ph | Pyrrolid | // | 258-259 |
| 143 | 6-F | Et | Ph | N(Me)Et | // | 174-175 |
| 144 | 6-$OCH_2Ph$ | Me | Ph | Pyrrolid | // | 199-201 |
| 145 | 6-$CF_3$ | Me | Ph | Pyrrolid | // | 211-212 |
| 146 | 6-F | H | Ph | Pyrrolid | // | 283-285 |

| N° | X | $R_1$ | $R_2$ | $NR_3R_4$ | 3-4 | F (°C) |
|---|---|---|---|---|---|---|
| 147 | 6-F | Me | Ph | $NH(CH_2)_4OH$ | // | 210-213 |
| 148 | 6-F | Me | Ph | $NH(CH_2)_3CO_2H$ | // | 278-279 |
| 149 | 6-F | Me | Ph | $NH(CH_2)_3CO_2Et$ | // | 154-156 |

<u>Légende</u>

Me désigne un groupe méthyle, Et désigne un groupe éthyle, Pr désigne un groupe propyle, iPr désigne un groupe isopropyle, cPr désigne un groupe cyclopropyle, Ph désigne un groupe phényle, 1-Napht et 2-Napht désignent, respectivement, des groupes naphtalén-1-yle et naphtalén-2-yle, x-Pyridyl désigne un groupe pyridin-x-yle, x-Thienyl désigne un groupe thién-x-yle, Piperid désigne un groupe pipéridin-1-yle, Pyrrolid désigne un groupe pyrrolidin-1-yle, Morph désigne un groupe morpholin-4-yle, Azetid désigne un groupe azétidin-1-yle, 4-Me-piperaz désigne un groupe 4-méthylpipérazin-1-yle, 5-Me-thiadiaz désigne un groupe 5-méthyl-1,3,4-thiadiazol-2-yle, 5-Me-oxazol désigne un groupe 5-méthyl-1,2-oxazol-2-yle, Thiazolid désigne un groupe thiazolidinyle.

Dans la colonne "3-4", "/" désigne une liaison carbone-carbone simple et "//" désigne un liaison carbone-carbone double entre les atome 3 et 4 de la molécule.

Dans la colonne "F (°C)", "d" désigne un point de fusion avec décomposition.

Les composés de l'invention ont été soumis à des essais pharmacologiques qui ont mis en évidence leur intérêt comme substances à activités thérapeutiques.

<u>Etude des liaisons membranaires vis-à-vis des récepteurs $\omega_1$ (benzodiazépiniques de type 1) et $\omega_2$ (benzodiazépiniques de type 2)</u>.

L'affinité des composés pour les récepteurs $\omega_1$ du cervelet et $\omega_2$ de la moëlle épinière a été déterminée selon une variante de la méthode décrite par S. Z. Langer et S. Arbilla dans *Fund. Clin. Pharmacol.,* **2**, 159-170 (1988), avec utilisation de $^3$H-flumazenil au lieu de $^3$H-diazepam comme radioligand.
On homogénéise le tissu du cervelet ou de la moëlle épinière pendant 60 s dans 120 ou 30 volumes, respectivement, de tampon glacé (50 mM Tris/HCl, pH 7,4, 120 mM NaCl, 5 mM KCl) puis, après dilution à 1/3, on fait incuber la suspension avec du $^3$H-flumazenil (activité spécifique 78 Ci/mmole, New England Nuclear) à une concentration de 1 nM et avec les composés de l'invention à différentes concentrations, dans un volume final de 525 µl. Après 30 minutes d'incubation à 0°C on filtre les échantillons sous pression réduite sur des filtres Whatman GF/B® et on les lave immédiatement avec du tampon glacé. La liaison spécifique du $^3$H-flumazenil est déterminée en présence de diazépam 1

µM non marqué. On analyse les données selon les méthodes usuelles et on calcule la concentration $CI_{50}$, concentration qui inhibe de 50% la liaison du $^3$H-flumazenil.
Les $CI_{50}$ des composés de l'invention les plus actifs se situent, dans cet essai, entre 10 et 1000 nM.

Etude de l'activité anxiolytique : test du conflit de prise de boisson.

L'activité anxiolytique est évaluée chez le rat dans le test de conflit de prise de boisson, selon la méthode décrite par J. R. Vogel, B. Beer et D. E. Clody dans *Psychopharmacologia* (Berl.), **21**, 1-7 (1971).
Après une diète hydrique de 48h, le rat est placé dans une enceinte isolée du bruit et équipée d'une pipette d'eau reliée à un anxiomètre délivrant un léger choc électrique tous les 20 coups de langue. Le nombre de chocs reçus est automatiquement compté pendant 3 minutes, et permet d'évaluer l'activité anxiolytique des composés testés. Les résultats sont exprimés par la dose efficace minimale (DEM), dose qui produit une augmentation significative du nombre de chocs reçus, par rapport au nombre observé chez les animaux témoins.
Les DEM des composés les plus actifs se situent, dans cet essai, entre 5 et 50 mg/kg par la voie intrapéritonéale.

Etude de l'activité anxiolytique : test du labyrinthe surélevé en croix.

Le protocole de cet essai est une modification de celui décrit par S. Pellow et S. File dans *Pharmacol. Biochem. Behav.* (1986) **24** 525-529.
Après une période d'habituation à la pièce expérimentale de 24 h environ, les rats sont placés individuellement sur la plate-forme centrale, le museau dirigé vers un des bras fermés, et observés pendant 4 min par l'intermédiaire d'une caméra vidéo. On enregistre le temps passé par l'animal dans les bras ouverts, le nombre d'entrées dans les bras fermés et dans les bras ouverts, le nombre de tentatives d'entrée dans les bras ouverts suivies par une réponse d'évitement et l'exploration des rebords dans les bras ouverts. Les résultats sont exprimés pour chaque animal : 1° en pourcentage de passages dans les bras ouverts par rapport au nombre total d'entrées dans les quatres bras de l'appareil, 2° en pourcentage de temps passé dans les bras ouverts par rapport à la durée totale du test (4 min), 3° en nombre total de tentatives avortées effectuées par l'animal, 4° en nombre total d'explorations.
Les produits à étudier sont administrés par voie intrapéritonéale ou orale à doses croissantes.
Les résultats sont exprimés par la dose efficace minimale (DEM) qui produit soit une augmentation significative (activité dans les bras ouverts) soit une diminution significative (tentatives) par rapport aux performances observées chez les animaux témoins.
Les DEM des composés les plus actifs se situent, dans cet essai, entre 3 et 50 mg/kg par la voie intrapéritonéale ou orale.

Etude de l'activité hypnotique.

L'activité sédative ou hypnotique des composés a été déterminée par l'observation de leur action sur l'électrocorticogramme du rat, selon la méthode décrite par H. Depoortere, *Rev. E.E.G. Neurophysiol.,* **10**, 3, 207-214 (1980) et par H. Depoortere et M. Decobert, *J. Pharmacol. (Paris)*, **14**, 2, 195-265 (1983).
Les produits à étudier ont été administrés par voie intrapéritonéale à doses croissantes. Ils induisent des tracés de sommeil à des doses allant de 1 à 30 mg/kg.

Etude de l'activité anticonvulsivante : activité vis-à-vis des convulsions cloniques induites chez le rat par injection de pentétrazol.

Le protocole de cet essai est une modification de celui décrit par E. A. Swinyard et J. H. Woodhead dans *Antiepileptic Drugs,* Raven Press, New York, 111-126 (1982). Les produits à tester sont administrés aux animaux par voie intrapéritonéale 30 min avant une injection intraveineuse de 20 mg/kg de pentétrazol. Immédiatement après l'injection on note pendant 5 min le nombre d'animaux présentant des convulsions cloniques.
Les résultats sont exprimés par la $DA_{50}$, dose qui protège 50% des animaux, calculée selon la méthode de J. T. Lichtfield et F. Wilcoxon (*J. Pharm. Exp. Ther.* (1949) **96** 99-113) à partir de 3 ou 4 doses administrées chacune à un groupe de 8 à 10 rats.
Les $DA_{50}$ des composés les plus actifs se situent entre 0,3 et 30 mg/kg par la voie intrapéritonéale ou orale.

Etude de l'activité anticonvulsivante : activité vis-à-vis des convulsions induites chez la souris par l'isoniazide.

L'activité intrinsèque des composés est déterminée par le temps de latence d'apparition des convulsions induites par l'administration sous-cutanée d'isoniazide (800 mg/kg) simultanément avec le composé à tester, injecté

par voie intrapéritonéale, selon le protocole décrit par G. Perrault, E. Morel, D. Sanger et B. Zivkovic dans *Eur. J. Pharmacol.,* **156**, 189-196 (1988). Les résultats sont exprimés par la $DA_{50}$, dose qui produit 50% de l'effet maximal, par rapport aux animaux témoins, déterminée à partir de 3 ou 4 doses administrées chacune à un groupe de 8 à 10 souris.
Les $DA_{50}$ des composés de l'invention se situent, dans cet essai, entre 1 et 50 mg/kg par la voie intrapéritonéale et, selon les composés, l'effet maximal peut aller jusqu'à 300%.

Les résultats des essais effectués sur les composés de l'invention montrent que, *in vitro,* ils déplacent le $^3$H-flumazénil de ses sites de liaison spécifiques au niveau du cervelet et de la moëlle épinière ; ils présentent une affinité mixte pour les sites $\omega_1$ et $\omega_2$ (benzodiazépiniques de type 1 et de type 2) situés au sein du complexe macromoléculaire $GABA_A$-sites ω-canal chlorure.
*In vivo* ils se comportent comme des agonistes, complets ou partiels, vis-à-vis de ces récepteurs.
Ils possèdent des propriétés anxiolytiques, hypnotiques et anticonvulsivantes et, par conséquent, peuvent être utilisés pour le traitement d'affections liées aux désordres de la transmission GABAergique, tels que l'anxiété, les troubles du sommeil, l'épilepsie, la spasticité, les contractures musculaires, les troubles cognitifs, les troubles du sevrage vis-à-vis de l'alcoolisme, du tabac, des stupéfiants, etc. Ils peuvent également être utilisés pour le traitement de la maladie de Parkinson ainsi que de tous types de syndromes extrapyramidaux.
Enfin, ils peuvent être utilisés dans la prémédication et comme anesthésiques généraux pour l'induction et/ou le maintien de l'anesthésie, ou comme anesthésiques locaux, éventuellement -associés à d'autres anesthésiques et/ou des myorelaxants et/ou des analgésiques.

A cet effet ils peuvent être présentés sous toutes formes galéniques, associés à des excipients appropriés, pour l'administration entérale ou parentérale, par exemple sous forme de comprimés, dragées, gélules, capsules, solutions ou suspensions buvables ou injectables, suppositoires, etc, dosés pour permettre une administration journalière de 1 à 1000 mg de substance active.

## Revendications

1. Composé, éventuellement sous forme d'isomère optique pur ou de mélange de tels isomères, répondant à la formule générale (I)

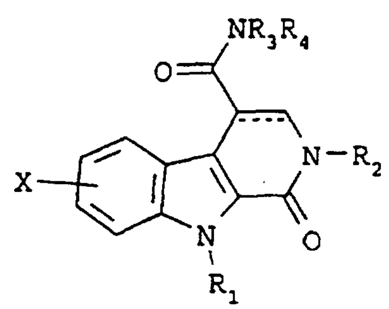

(I)

dans laquelle

X représente un atome d'hydrogène ou d'halogène ou un groupe $(C_1-C_3)$alkyle, $(C_1-C_3)$alcoxy, trifluorométhyle ou phénylméthoxy,
$R_1$ représente un atome d'hydrogène ou un groupe $(C_1-C_3)$-alkyle, cyclopropylméthyle ou phénylméthyle,
$R_2$ représente soit un groupe $(C_1-C_3)$alkyle éventuellement substitué par un groupe méthoxy, soit un groupe phényl-$(C_1-C_3)$alkyle éventuellement substitué sur le noyau phényle par un atome d'halogène ou un groupe méthyle ou méthoxy, soit un groupe cyclohexylméthyle, soit un groupe thiénylméthyle, soit un groupe pyridinylméthyle, soit un groupe phényle éventuellement substitué par un ou plusieurs atomes d'halogène ou un groupe $(C_1-C_3)$alkyle ou $(C_1-C_3)$alcoxy, soit un groupe pyridinyle, soit un groupe 5-méthyl-1,2-oxazolyle, soit un groupe 5-méthyl-1,3,4-thiadiazolyle, soit un groupe naphtalényle,
$R_3$ et $R_4$, indépendamment l'un de l'autre, représentent chacun un atome d'hydrogène, un groupe $(C_1-C_3)$ alkyle, un groupe 2-méthoxyéthyle, un groupe hydroxy$(C_2-C_4)$alkyle, un groupe carboxy$(C_1-C_3)$alkyle, un groupe $(C_1-C_3)$alcoxycarbonyl $(C_1-C_3)$-alkyle ou un groupe phényl$(C_1-C_3)$alkyle, ou bien forment ensemble, avec l'atome d'azote qui les porte, soit un groupe pyrrolidinyle éventuellement substitué par un groupe hydroxy, éthoxy, méthoxycarbonyle ou méthoxyméthyle, soit un groupe pipéridinyle, soit un groupe morpholinyle, soit

un groupe 4-méthylpipérazinyle, soit un groupe azétidinyle, soit un groupe thiazolidinyle, et la liaison entre les atomes de carbone en positions 3 et 4 est simple ou double.

2. Composé selon la revendication 1, **caractérisé en ce que**, dans la formule générale (I), X est en position 6 et représente un atome de fluor.

3. Composé selon la revendication 1, **caractérisé en ce que** $R_1$ représente un groupe méthyle.

4. Composé selon la revendication 1, **caractérisé en ce que** $R_2$ représente un groupe phényle.

5. Composé selon la revendication 1, **caractérisé en ce que** $R_3$ représente un groupe méthyle et $R_4$ représente un groupe éthyle.

6. Composé selon la revendication 1, **caractérisé en ce que** $R_3$ et $R_4$ forment, avec l'atome d'azote qui les porte, un cycle pyrrolidinyle.

7. Médicament **caractérisé en ce qu'**il est constitué d'un composé selon l'une des revendications 1 à 6.

8. Composition pharmaceutique **caractérisée en ce qu'**elle contient un composé selon l'une des revendications 1 à 6, associé à un excipient.

9. Procédé de préparation d'un composé selon la revendication 1, **caractérisé en ce qu'**on fait réagir un composé de formule générale (II)

;

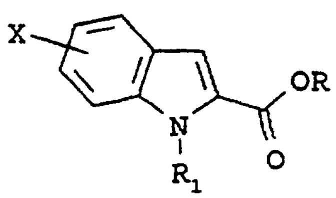

(II)

dans laquelle X et $R_1$ sont tels que définis dans la revendication 1 et R représente un groupe $(C_1-C_3)$alkyle, avec le pyruvate d'éthyle pour obtenir le diester de formule générale (IV)

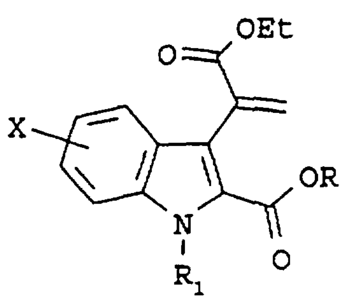

(IV)

puis on traite ce dernier avec une amine de formule générale $R_2NH_2$, dans laquelle $R_2$ est tel que défini dans la revendication 1, pour obtenir un ester de formule générale (V)

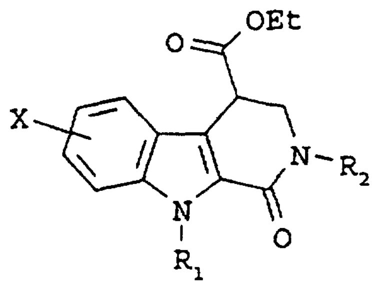

(V)

qu'on transforme en l'acide correspondant, de formule générale (VI)

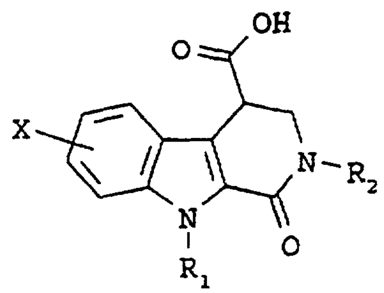

(VI)

par hydrolyse, puis on transforme cet acide en amide primaire, secondaire ou tertiaire de formule générale (I')

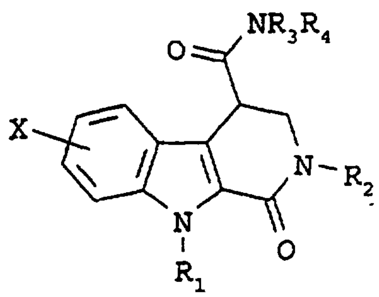

(I')

par action d'une amine de formule générale $HNR_3R_4$, dans laquelle $R_3$ et $R_4$ sont tels que définis dans la revendication 1, soit en passant par l'imidazolide obtenu par action du *N,N'*-carbonyldiimidazole, soit en passant par le chlorure d'acide, et enfin, si l'on désire préparer un composé dans lequel la liaison entre les positions 3 et 4 est une liaison double, on soumet le composé de formule générale (I') à une oxydation au moyen de 2,3-dichloro-5,6-dicyanocyclohexa-2,5-diène-1,4-dione ou de 3,4,5,6-tétrachlorocyclohexa-3,5-diène-1,2-dione pour obtenir le composé correspondant, de formule qénérale (I")

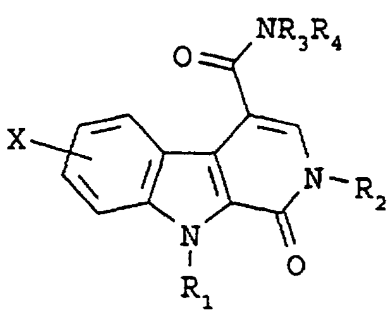

(I")

**10.** Procédé de préparation d'un composé selon la revendication 1, **caractérisé en ce qu'**on fait réagir un composé de formule générale (VIII)

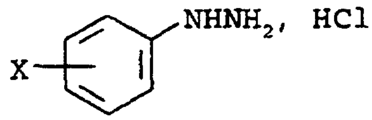

(VIII)

dans laquelle X est tel que défini dans la revendication 1, avec l'acide 2-cétoglutarique, puis on le traite dans un milieu alcoolique acide, pour obtenir le diester de formule générale (IX)

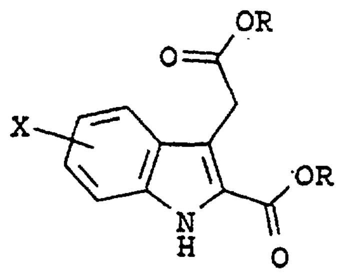

(IX)

dans laquelle R représente un groupe ($C_1$-$C_3$) alkyle, puis, si on le désire, on effectue une réaction d'alkylation pour obtenir le composé de formule générale (X)

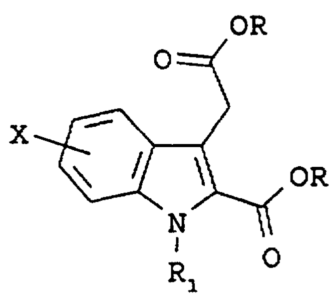

(X)

dans laquelle $R_1$ représente un groupe ($C_1$-$C_3$) alkyle, puis on transforme ce dernier en présence de diméthylacétaldiméthylformamide pour obtenir le composé de formule générale (XI)

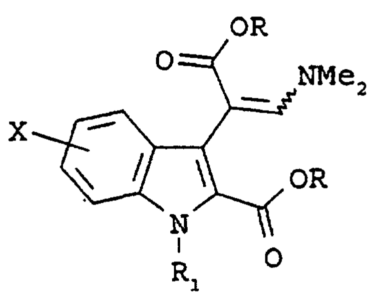

(XI)

ou bien, si on le désire, on transforme directement le composé de formule générale (IX) en composé de formule générale (XI) dans laquelle $R_1$ représente un groupe méthyle, puis on traite ce dernier avec une amine de formule générale $H_2NR_2$, dans laquelle $R_2$ est tel que défini dans la revendication 1, pour obtenir l'ester de formule générale (V')

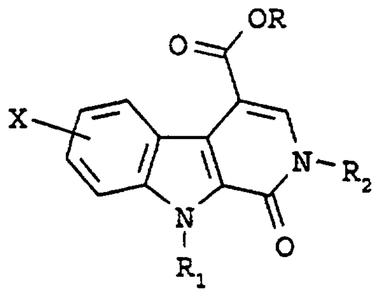

(V')

puis on transforme ce dernier en acide correspondant, de formule générale (VI')

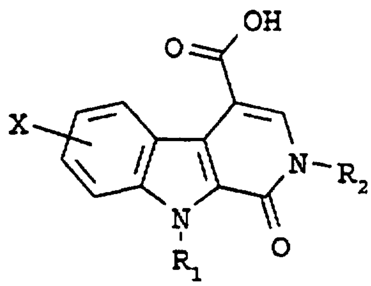

(VI')

et enfin on transforme cet acide en amide primaire, secondaire ou tertiaire de formule générale (I")

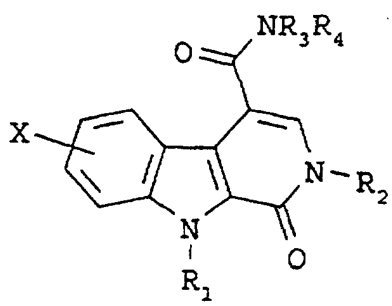

(I")

par action d'une amine de formule générale $HNR_3R_4$, dans laquelle $R_3$ et $R_4$ sont tels que définis dans la revendication 1,
soit en passant par l'imidazolide obtenu par action du *N,N'*-carbonyldimidazole, soit en passant par le chlorure d'acide.

**11.** Procédé de préparation d'un composé selon la revendication 1, **caractérisé en ce qu'**on hydrolyse un diester de formule générale (X)

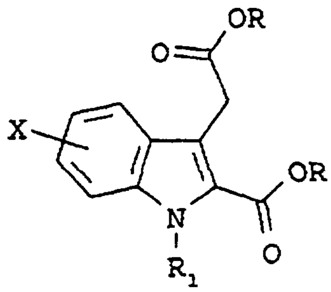

(X)

dans laquelle X et $R_1$ sont tels que décrits dans la revendication 1 et R représente un groupe ($C_1$-$C_3$) alkyle, pour obtenir le diacide de formule générale (XII)

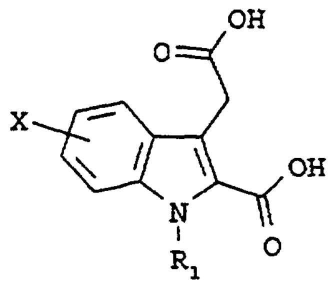

(XII)

que l'on transforme en anhydride pour obtenir le composé de formule générale (XIII)

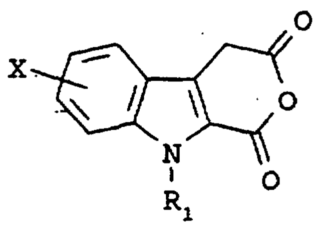

(XIII)

puis on transforme ce dernier par action d'une amine de formule générale $HNR_3R_4$, dans laquelle $R_3$ et $R_4$ sont tels que définis dans la revendication 1, pour obtenir le composé de formule générale (XIV)

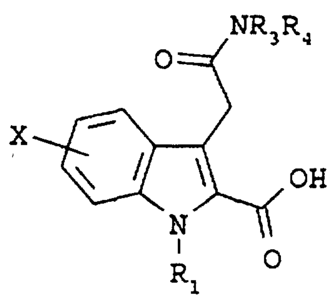

(XIV)

que l'on transforme en ester de formule générale (XV)

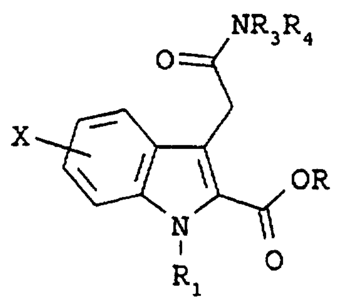

(XV)

puis on traite ce dernier en présence de diméthylacétal diméthylformamide pour obtenir le composé de formule générale (XVI)

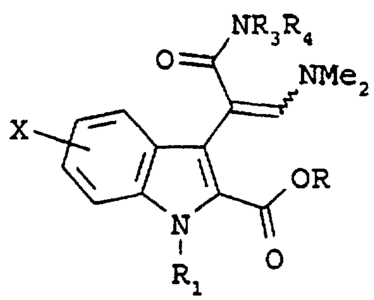

(XVI)

et finalement on fait réagir ce dernier avec une amine de formule générale $R_2NH_2$, dans laquelle $R_2$ est tel que défini dans la revendication 1, pour obtenir le composé de formule générale (I")

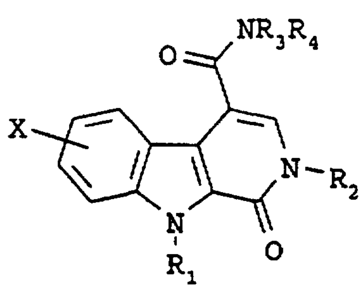

(I")

et enfin, si on le désire, on transforme un amide secondaire de formule générale (I") en amide tertiaire par une réaction d'alkylation.

## Patentansprüche

**1.** Verbindung, gegebenenfalls in Form des reinen optischen Isomeren oder einer Mischung solcher Isomeren, der allgemeinen Formel (I)

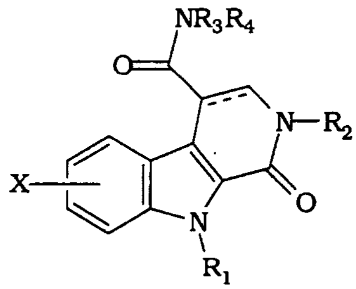

(I)

in der

X ein Wasserstoffatom, ein Halogenatom oder eine ($C_1$-$C_3$)-Alkylgruppe, ($C_1$-$C_3$)-Alkoxygruppe, Trifluormethylgruppe oder Phenylmethoxygruppe.
$R_1$ ein Wasserstoffatom oder eine ($C_1$-$C_3$)-Alkylgruppe, Cyclopropylmethylgruppe oder Phenylmethylgruppe,
$R_2$ entweder eine ($C_1$-$C_3$)-Alkylgruppe, die gegebenenfalls durch eine Methoxygruppe substituiert ist, oder eine Phenyl-($C_1$-$C_3$)-alkylgruppe, die gegebenenfalls am Phenylkern durch ein Halogenatom oder eine Methyl- oder Methoxygruppe substituiert ist, oder eine Cyclohexylmethylgruppe, oder eine Thienylmethylgruppe, oder eine Pyridinylmethylgruppe, oder eine Phenylgruppe, die gegebenenfalls durch ein oder mehrere Halogenatome oder eine ($C_1$-$C_3$)-Alkylgruppe oder ($C_1$-$C_3$)-Alkoxygrupppe substituiert ist, oder eine Pyridinylgruppe, oder eine 5-Methyl-1,2-oxazolylgruppe, oder eine 5-Methyl-1,3,4-thiadiazolylgruppe, oder eine Naphthalinylgruppe,
$R_3$ und $R_4$ unabhängig voneinander jeweils ein Wasserstoffatom, eine ($C_1$-$C_3$)-Alkylgruppe, eine 2-Methoxyethylgruppe, eine Hydroxy-($C_2$-$C_4$)-alkylgruppe, eine Carboxy-($C_1$-$C_3$)-Alkylgruppe, eine ($C_1$-$C_3$)-Alkoxycarbonyl-($C_1$-$C_3$)-alkylgruppe oder eine Phenyl-($C_1$-$C_3$)-alkylgruppe oder gemeinsam mit dem sie tragenden Stickstoffatom entweder eine Pyrrolidinylgruppe, die gegebenenfalls durch eine Hydroxygruppe, Ethoxygruppe, Methoxycarbonylgruppe oder Methoxymethylgruppe substituiert ist, oder eine Piperidinylgruppe, oder eine Morpholinylgruppe, oder eine 4-Methylpiperazinylgruppe, oder eine Azetidinylgruppe, oder eine Thiazolidinylgruppe, und
die Bindung zwischen den Kohlenstoffatomen in den 3- und 4-Stellungen eine Einfachbindung oder eine Doppelbindung bedeuten.

**2.** Verbindung nach Anspruch 1, **dadurch gekennzeichnet, daß** in der allgemeinen Formel (I) X in der 6-Stellung steht und ein Fluoratom bedeutet.

**3.** Verbindung nach Anspruch 1, **dadurch gekennzeichnet, daß** $R_1$ eine Methylgruppe bedeutet.

**4.** Verbindung nach Anspruch 1, **dadurch gekennzeichnet, daß** $R_2$ eine Phenylgruppe bedeutet.

**5.** Verbindung nach Anspruch 1, **dadurch gekennzeichnet, daß** R3 eine Methylgruppe und $R_4$ eine Ethylgruppe bedeuten.

**6.** Verbindung nach Anspruch 1, **dadurch gekennzeichnet, daß** $R_3$ und $R_4$ gemeinsam mit dem sie tragenden Stickstoffatom einen Pyrrolidinylring bilden.

**7.** Arzneimittel, **dadurch gekennzeichnet, daß** es durch eine Verbindung nach einem der Ansprüche 1 bis 6 gebildet wird.

**8.** Pharmazeutische Zubereitung, **dadurch gekennzeichnet, daß** sie eine Verbindung nach einem der Ansprüche 1 bis 6 in Kombination mit einem Trägermaterial enthält.

**9.** Verfahren zur Herstellung einer Verbindung nach Anspruch 1, **dadurch gekennzeichnet, daß** man eine Verbindung der allgemeinen Formel (II)

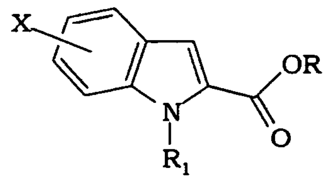

(II)

in der X und $R_1$ die in Anspruch 1 angegebenen Bedeutungen besitzen und R eine ($C_1$-$C_3$)-Alkylgruppe darstellt, mit Brenztraubensäureethylester umsetzt zur Bildung des Diesters der allgemeinen Formel (IV)

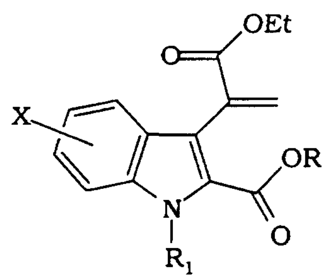

(IV)

man diesen letzteren mit einem Amin der allgemeinen Formel $R_2NH_2$, worin $R_2$ die in Anspruch 1 angegebenen Bedeutungen besitzt, behandelt zur Bildung eines Esters der allgemeinen Formel (V)

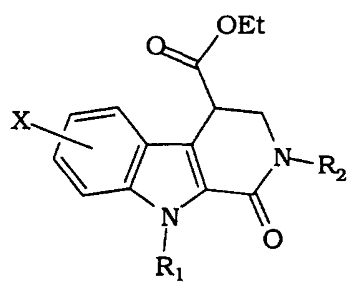

(V)

welchen man durch Hydrolyse in die entsprechende Säure der allgemeinen Formel (VI)

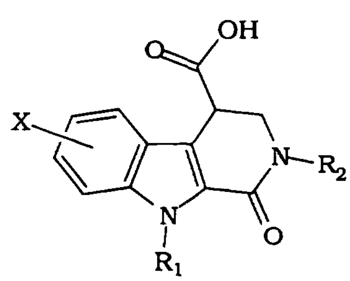

(VI)

umwandelt und dann diese Säure in das primäre, sekundäre oder tertiäre Amid der allgemeinen Formel (I') umwandelt

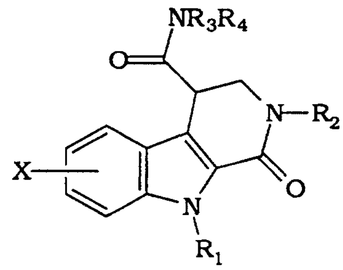

(I')

durch Umsetzen mit einem Amin der allgemeinen Formel $HNR_3R_4$, in der $R_3$ und $R_4$ die in Anspruch 1 angegebenen Bedeutungen besitzen, oder über das durch Einwirkung von *N,N'*-Carbonyldiimidazol erhaltene Imidazolid, oder über das Säurechlorid, und schließlich, wenn eine Verbindung hergestellt werden soll, bei der die Bindung zwischen den Stellungen 3 und 4 eine Doppelbindung darstellt, man die Verbindung der allgemeinen Formel (I') einer Oxidation mit 2,3-Dichlor-5,6-dicyanocyclohexa-2,5-dien-1,4-dion oder 3,4,5,6-Tetrachlorcyclohexa-3,5-dien-1,2-dion unterwirft zur Bildung der entsprechenden Verbindung der allgemeinen Formel (I")

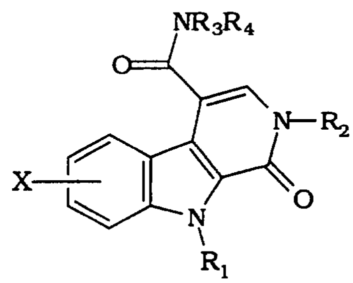

(I")

**10.** Verfahren zur Herstellung einer Verbindung nach Anspruch 1, **dadurch gekennzeichnet, daß** man eine Verbindung der allgemeinen Formel (VIII)

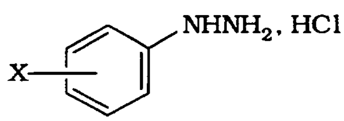

(VIII)

in der X die in Anspruch 1 angegebenen Bedeutungen besitzt, mit 2-Ketoglutarsäure umsetzt und dann in einem alkoholischen sauren Medium behandelt zur Bildung des Diesters der allgemeinen Formel (IX)

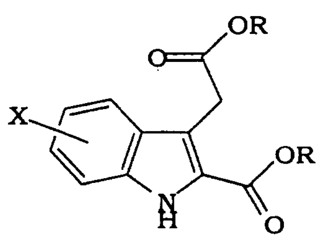

(IX)

in der R eine ($C_1$-$C_3$)-Alkylgruppe bedeutet, und man dann gewünschtenfalls eine Alkylierungsreaktion durchführt zur Bildung der Verbindung der allgemeinen Formel (X)

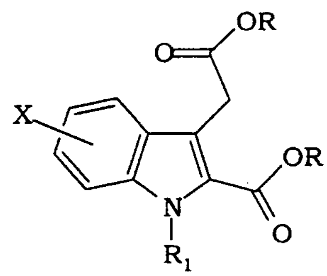

(X)

in der $R_1$ eine $(C_1-C_3)$-Alkylgruppe darstellt, wonach man diese letztere Verbindung in Gegenwart von Dimethylacetaldimethylformamid umwandelt zur Bildung der Verbindung der allgemeinen Formel (XI)

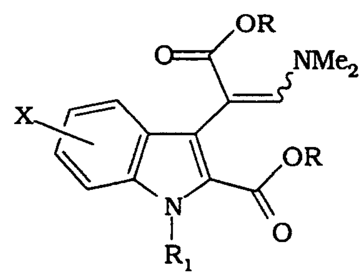

(XI)

oder man gewünschtenfalls die Verbindung der allgemeinen Formel (IX) direkt in die Verbindung der allgemeinen Formel (XI) umwandelt, in der $R_1$ eine Ethylgruppe bedeutet, worauf man diese letztere Verbindung mit einem Amin der allgemeinen Formel $H_2NR_2$, in der $R_2$ die in Anspruch 1 angegebenen Bedeutungen besitzt, behandelt zur Bildung des Esters der allgemeinen Formel (V')

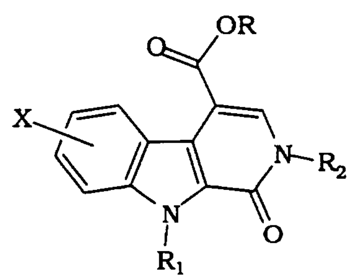

(V')

und man diesen letzteren in die entsprechende Säure der allgemeinen Formel (VI') umwandelt

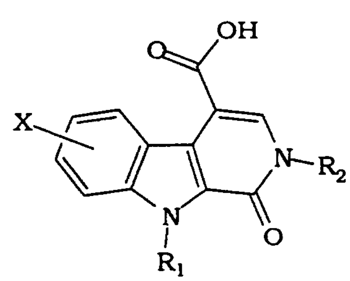

(VI')

oder man schließlich diese Säure in das primäre, sekundäre oder tertiäre Amid der allgemeinenFormel (I")

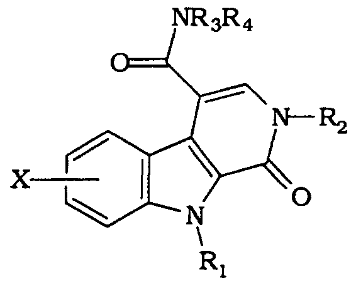

(I")

umwandelt durch Umsetzen mit einem Amin der allgemeinen Formel $HNR_3R_4$, in der $R_3$ und $R_4$ die in Anspruch 1 angegebenen Bedeutungen besitzen, oder indem man die Reaktion über das durch Einwirkung von *N,N'*-Carbonyldiimidazol erhaltene Imidazolid oder über das Säurechlorid führt.

**11.** Verfahren zur Herstellung einer Verbindung nach Anspruch 1, **dadurch gekennzeichnet, daß** man einen Diester der allgemeinen Formel (X)

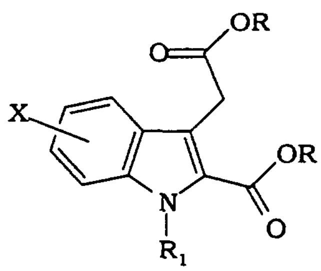

(X)

in der X und $R_1$ die in Anspruch 1 angegebenen Bedeutungen besitzen und R eine ($C_1$-$C_3$)-Alkylgruppe darstellt, hydrolysiert zur Bildung der Disäure der allgemeinen Formel (XII)

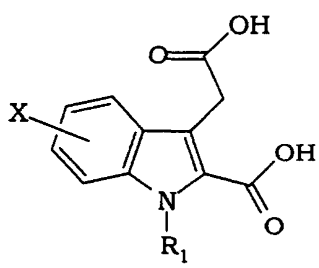

(XII)

welche man in das Anhydrid umwandelt zur Bildung der Verbindung der allgemeinen Formel (XIII)

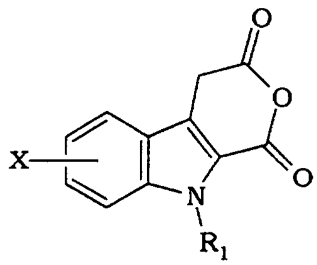

(XIII)

und man dann dieses letztere durch Umsetzen mit einem Amin der allgemeinen Formel $HNR_3R_4$, worin $R_3$ und $R_4$ die in Anspruch 1 angegebenen Bedeutungen besitzen, in die Verbindung der allgemeinen Formel (XIV) umwandelt

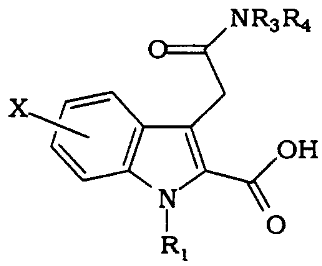

(XIV)

welche man in einen Ester der allgemeinen Formel (XV) umwandelt

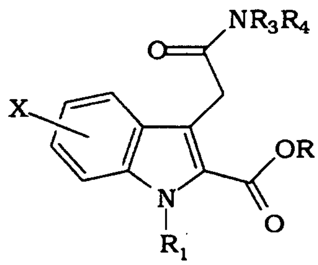

(XV)

und man dann diesen letzteren mit Dimethylacetaldimethylformamid behandelt zur Bildung der Verbindung der allgemeinen Formel (XVI)

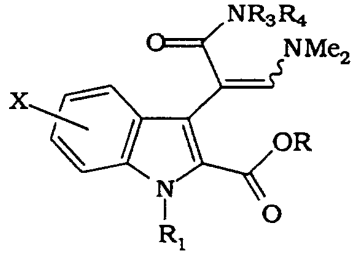

(XVI)

und man schließlich diese letztere Verbindung mit einem Amin der allgemeinen Formel $R_2NH_2$, worin $R_2$ die in Anspruch 1 angegebenen Bedeutungen besitzt, umsetzt zur Bildung der Verbindung der allgemeinen Formel (I")

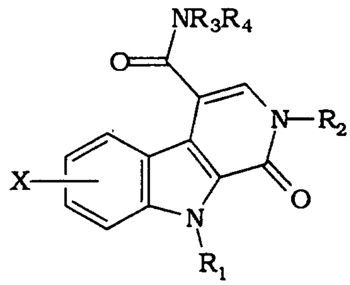

(I")

und man schließlich gewünschtenfalls ein sekundäres Amid der allgemeinen Formel (I") durch eine Alkylierungsreaktion in ein tertiäres Amid umwandelt.

## Claims

1. Compound, optionally in the form of a pure optical isomer or of a mixture of such isomers, corresponding to the general formula (I)

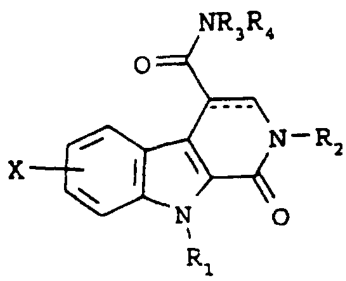

(I)

in which

X represents a hydrogen or halogen atom or a $(C_1-C_3)$alkyl, $(C_1-C_3)$alkoxy, trifluoromethyl or phenylmethoxy group,
$R_1$ represents a hydrogen atom or a $(C_1-C_3)$alkyl, cyclopropylmethyl or phenylmethyl group,
$R_2$ represents either a $(C_1-C_3)$alkyl group optionally substituted by a methoxy group, or a phenyl$(C_1-C_3)$alkyl group optionally substituted on the phenyl ring by a halogen atom or a methyl or methoxy group, or a cyclohexylmethyl group, or a thienylmethyl group, or a pyridinylmethyl group, or a phenyl group optionally substituted by one or more halogen atoms or a $(C_1-C_3)$alkyl or $(C_1-C_3)$alkoxy group, or a pyridinyl group, or a 5-methyl-1,2-oxazolyl group, or a 5-methyl-1,3,4-thiadiazolyl group, or a naphthyl group,
$R_3$ and $R_4$, independently of one another, each represent a hydrogen atom, a $(C_1-C_3)$alkyl group, a 2-methoxyethyl group, a hydroxy$(C_2-C_4)$alkyl group, a carboxy-$(C_1-C_3)$alkyl group, a $(C_1-C_3)$alkoxycarbonyl$(C_1-C_3)$ alkyl group or a phenyl$(C_1-C_3)$alkyl group, or else together form, with the nitrogen atom which carries them, either a pyrrolidinyl group optionally substituted by a hydroxyl, ethoxy, methoxycarbonyl or methoxymethyl group, or a piperidinyl group, or a morpholinyl group, or a 4-methylpiperazinyl group, or an azetidinyl group, or a thiazolidinyl group, and
the bond between the carbon atoms in the 3 and 4 positions is single or double.

2. Compound according to Claim 1, **characterized in that**, in the general formula (I), X is in the 6 position and represents a fluorine atom.

3. Compound according to Claim 1, **characterized in that** $R_1$ represents a methyl group.

4. Compound according to Claim 1, **characterized in that** $R_2$ represents a phenyl group.

**5.** Compound according to Claim 1, **characterized in that** $R_3$ represents a methyl group and $R_4$ represents an ethyl group.

**6.** Compound according to Claim 1, **characterized in that** $R_3$ and $R_4$ form, with the nitrogen atom which carries them, a pyrrolidinyl ring.

**7.** Medicament, **characterized in that** it is composed of a compound according to one of Claims 1 to 6.

**8.** Pharmaceutical composition, **characterized in that** it contains a compound according to one of Claims 1 to 6, in combination with an excipient.

**9.** Process for the preparation of a compound according to Claim 1, **characterized in that** a compound of general formula (II)

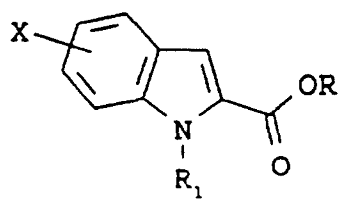

(II)

in which X and $R_1$ are as defined in Claim 1 and R represents a $(C_1\text{-}C_3)$alkyl group, is reacted with ethyl pyruvate, in order to obtain the diester of general formula (IV)

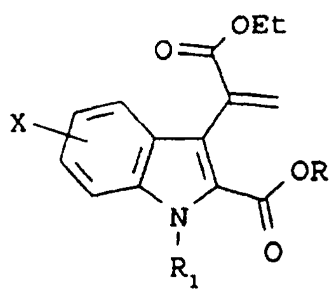

(IV)

the latter is then treated with an amine of general formula $R_2NH_2$, in which $R_2$ is as defined in Claim 1, in order to obtain an ester of general formula (V)

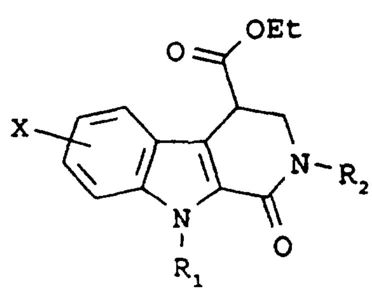

(V)

which is converted to the corresponding acid, of general formula (VI)

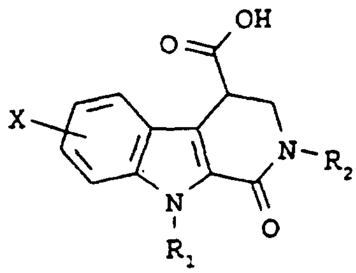

(VI)

by hydrolysis, this acid is then converted to the primary, secondary or tertiary amide of general formula (I')

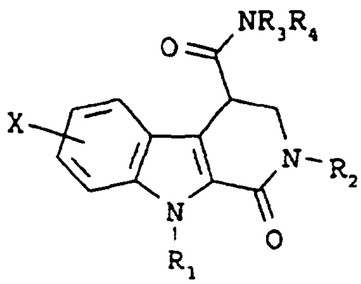

(I')

by reaction with an amine of general formula $HNR_3R_4$, in which $R_3$ and $R_4$ are as defined in Claim 1, either through the imidazolide obtained by reaction with *N,N'*-carbonyldiimidazole or through the acid chloride, and, finally, if it is desired to prepare a compound in which the bond between the 3 and 4 positions is a double bond, the compound of general formula (I') is oxidized by means of 2,3-dichloro-5,6-dicyanocyclohexa-2,5-diene-1,4-dione or of 3,4,5,6-tetrachlorocyclohexa-3,5-diene-1,2-dione, in order to obtain the corresponding compound of general formula (I'')

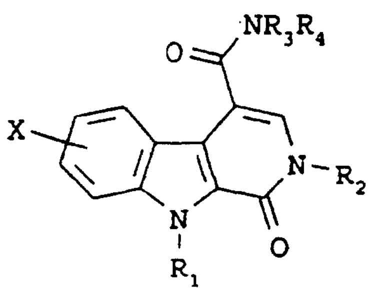

(I'')

**10.** Process for the preparation of a compound according to Claim 1, **characterized in that** a compound of general formula (VIII)

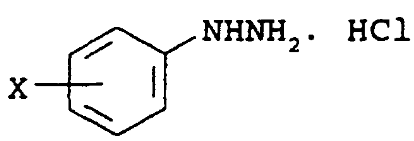

(VIII)

in which X is as defined in Claim 1, is reacted with 2-ketoglutaric acid, it is then treated in an acidic alcoholic medium, in order to obtain the diester of general formula (IX)

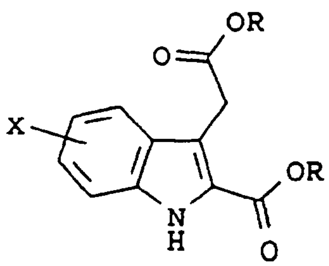

(IX)

in which R represents a $(C_1-C_3)$alkyl group, then, if desired, an alkylation reaction is carried out, in order to obtain the compound of general formula (X)

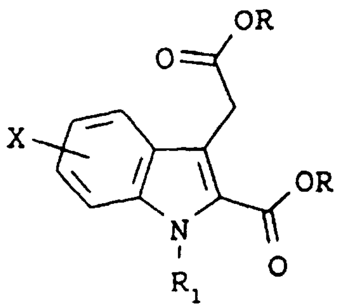

(X)

in which $R_1$ represents a $(C_1-C_3)$alkyl group, then the latter is converted in the presence of dimethylformamide dimethyl acetal, in order to obtain the compound of general formula (XI)

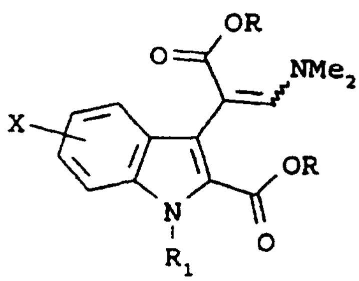

(XI)

or alternatively, if desired, the compound of general formula (IX) is directly converted to the compound of general formula (XI), in which $R_1$ represents a methyl group, the latter is then treated with an amine of general formula $H_2NR_2$, in which $R_2$ is as defined in Claim 1, in order to obtain the ester of general formula (V')

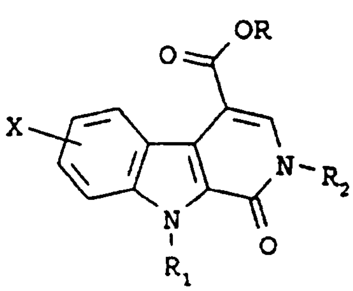

(V')

the latter is then converted to the corresponding acid of general formula (VI')

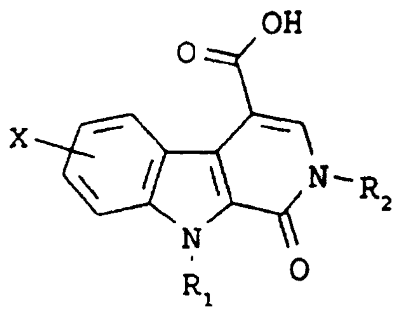

(VI')

and, finally, this acid is converted to the primary, secondary or tertiary amide of general formula (I")

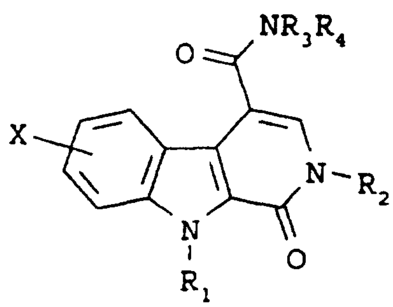

(I")

by reaction with an amine of general formula $HNR_3R_4$, in which $R_3$ and $R_4$ are as defined in Claim 1, either through the imidazolide obtained by reaction with *N,N'*-carbonyldiimidazole or through the acid chloride.

**11.** Process for the preparation of a compound according to Claim 1, **characterized in that** a diester of general formula (X)

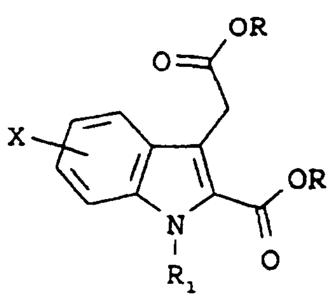

(X)

in which X and $R_1$ are as described in Claim 1 and R represents a $(C_1-C_3)$alkyl group, is hydrolysed, in order to obtain the diacid of general formula (XII)

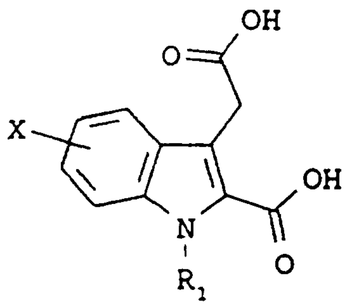

(XII)

which is converted to the anhydride, in order to obtain the compound of general formula (XIII)

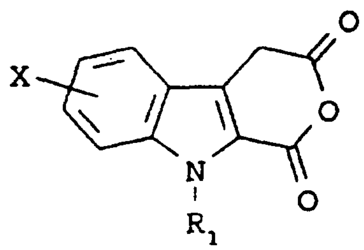

(XIII)

the latter is then converted by reaction with an amine of general formula $HNR_3R_4$, in which $R_3$ and $R_4$ are as defined in Claim 1, in order to obtain the compound of general formula (XIV)

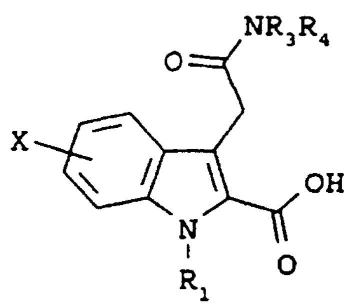

(XIV)

which is converted to the ester of general formula (XV)

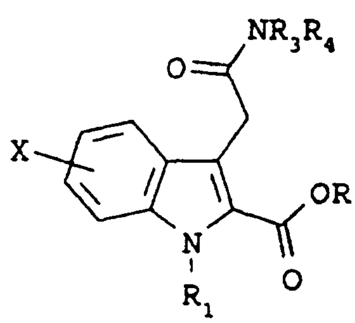

(XV)

the latter is then treated in the presence of dimethylformamide dimethyl acetal, in order to obtain the compound of general formula (XVI)

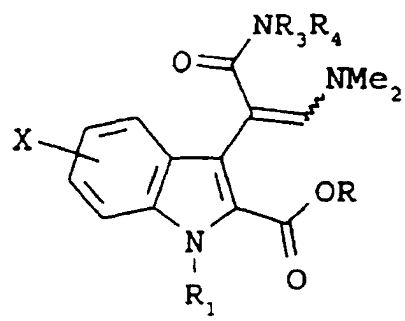

(XVI)

and, finally, the latter is reacted with an amine of general formula $R_2NH_2$, in which $R_2$ is as defined in Claim 1, in order to obtain the compound of general formula (I")

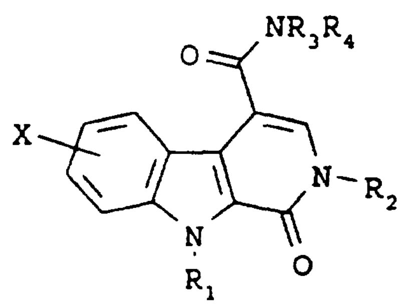

(I")

and, finally, if desired, a secondary amide of general formula (I") is converted to the tertiary amide by an alkylation reaction.